# EUROPEAN PATENT APPLICATION

(11) **EP 4 032 972 A1**
(43) Date of publication of application: **27.07.2022**
(21) Application number: 20864637.2
(22) Date of filing: 18.09.2020
(51) Int. Cl.: C12N 5/0783, C12N 9/10, C12N 9/78, C07K 14/495, C07K 14/54, C07K 14/725, C07K 16/28, A61K 35/17, A61K 39/00, A61K 45/06

(54) **GENETICALLY MODIFIED NK CELL LINE TRANSDUCED WITH GENE ENCODING NOVEL CHIMERIC ANTIGEN RECEPTOR AND USE THEREOF**

(30) Priority: 18.09.2019 KR 20190115034
(71) Applicant: Slbigen Inc., Seongnam-si, Gyeonggi-do 13488 (KR)
(72) Inventor: SUNG, Young Chul, Seoul 04419 (KR); KIM, Sae Won, Seoul 04593 (KR); HWANG, Injung, Seoul 04109 (KR)
(74) Representative: Dehns
(86) International application number: PCT/KR2020/012668
(87) International publication number: WO 2021/054789

(57) **Abstract**

The present invention provides a genetically modified NK cell line prepared by transducing host NK cell line with a gene construction encoding a cancer antigen-specific chimeric antigen receptor (CAR) comprising a FLT3-specific monoclonal antibody or a functional fragment thereof, a transmembrane domain, and a CD3ζ domain of a T-cell receptor for more efficient immunotherapy of acute myeloid leukemia, and use thereof, for more efficient immunotherapy of acute myeloid leukemia.

## Description

### TECHNICAL FIELD

The present invention relates to a genetically modified immune cell line transformed with a chimeric antigen receptor-encoding gene and uses thereof, and more particularly, to a novel chimeric antigen receptor-encoding gene transduced with improved anticancer activity, in particular, anticancer activity against acute myeloid leukemia. Genetically modified NK cell lines and uses thereof.

### BACKGROUND OF ART

Immune cell-based anti-cancer therapy is a treatment technology that suppresses and eliminates the proliferation of cancer cells by extracting and proliferating the cancer patient's own immune cells and then re-administering them to the patient. Among various types of immune cells, dendritic cells (DCs), natural killer cells (NKs) and cytotoxic T lymphocytes (CTLs) are mainly used for immune cell anti-cancer therapy. In particular, CTLs have the advantage of having an immunological memory function, antigen specificity, and excellent *in vivo* proliferative ability.

T cell therapeutics are being developed up to the 3^{rd} generation so far. The 1^{st} generation T cell therapeutics have low specificity for cancer cells and thus efficacy thereof could not be expected because they are administered to patients after proliferating all types of T cells (bulk T cells) present in the blood or cancer tissue. In addition, the 2^{nd} generation T cell therapeutics showed an improved therapeutic effect by isolating/mass-culturing only tumor antigen-specific T cells and administering them to cancer patients, whereas the cultivation period is prolonged and the complexity of the process has been raised. The 3^{rd} generation T cell therapeutics showed not only increased antigen specificity and shortened manufacturing time but also improved their therapeutic effect resulting in a remission rate close to 100% in some leukemias and lymphomas by using either 1) direct introducing a TCR gene that recognizes a specific cancer antigen into T cells, or 2) preparing a fusion protein by linking an antigen recognition site (scFv) of a monoclonal antibody that recognizes a specific antigen to T cell activation domain and then introducing it to T cells.

The 3^{rd} generation T cell therapeutics as described above are characterized by being genetically engineered to express a so-called chimeric antigen receptor (hereinafter, abbreviated as 'CAR'), but currently there is no CAR therapeutic approved by US FDA.

The companies leading the CAR-T technologies are Novartis, Juno Therapeutics, and Kite Pharma of the United States, all of which have developed CAR-T cells that target CD19, a B cell-specific antigen, and developed resistant/recurrent acute lymphoblastic lymphoma. It has been established as a leader in the field of targeted immune cell therapy, showing a high remission rate of close to 80 to 90% in acute lymphoblastic leukemia (ALL) and non-Hodgkin's lymphoma (NHL) (Hartmann et al., EMBO Mol. Med. 9(9): 1183-1197, 2017).

However, in the case of the CD19-targeted CAR-T cells, only ALL and NHL are indicated, so they have poor versatility and only target B cell-specific antigens. In recent clinical trials targeting acute myeloid leukemia (AML), the anticancer efficacy of CAR-T therapeutics is insignificant, while it is accompanied by serious side effects such as cytokine release syndrome. Thus, development of a next-generation CAR platform in order to overcome the side effects is urgently required.

On the other hand, natural killer cell lines are a commercially attractive quality-controlled immunotherapy platform with no modifications required, based on unlimited expandability, cell uniformity, product stability (especially after freeze/thaw) and batch-to-batch consistency. (Yang et al., J. Immunother. Cancer 7: 138, 2019) . Among them, NK-92 is the most intensively characterized NK cell line that has been approved for clinical trials by the FDA (Klingemann et al., Front. Immunol. 7: 91, 2016). Under GMP-compliant conditions, NK-92 cells can proliferate in a xeno-free, feeder-independent manner (Arai et al., Cytother. 10: 625-632, 2008). A therapeutic dose of up to 10⁹ cells per 1 L culture bag was reached within 3 weeks (Arai et al., Cytother. 10: 625-632, 2008). In cancer patients, repeated administration of NK-92 at doses of up to 5×10⁹ cells/m² every other day (three infusions at intervals of up to 6 months) has been shown to be safe and well tolerated (Williams et al., Oncotarget 8: 89256-68928, 2017), which has further increased its value as a platform for adoptive cell transfer. However, even at a high dose of NK-92 injection, it showed limited clinical efficacy as only 2 cases of complete remission were reported among 39 patients to be treated (Yang et al., J. Immunother. Cancer 7: 138, 2019). In addition, the clinical attractiveness of NK cell line-based therapy is further reduced because radiation is required before injection to patients and loss of persistence *in vivo* has been reported after administration (Zhang et al., Front. Immunol. 8: 533, 2017; Tang et al., Am. J. Cancer Res. 8: 1083-1089, 2018; Qu et al., MRS Commun. 9: 433-440, 2019). In response to these shortcomings, numerous efforts have been made to improve the function of NK cell lines, particularly through genetic engineering.

Transgenic strategies of NK cell lines can be divided into the following four categories, which are focused on enhancing tumor recognition and apoptotic activity: First, cytolytic activity of NK cell lines appeared to recover by introduction of an activating or expressing depleted co-stimulatory receptor. Liu et *al.* succeeded to induce surface expression of CD11a/CD18αβ heterodimer (LFA-1), resulting in increased cytotoxicity of NK cells to Raji cells. This was probably achieved by promoting LFA-1-mediated adhesion to tumor cells (Liu et al., Cell Immunol. 156: 24-35, 1994). Second, enhancement of effector function was caused by ectopic expression of cytokines in NK cell lines for autocrine stimulation. For example, IL-15 genetic modification induced increased tumor killing activity with IFN-γ secretion in both NKL and NK-92 (Jiang et al., Immunobiol. 219: 547-553, 2014; Zhang et al., Haematologica 89: 338-347, 2004). Third, as a measure to overcome the tumor microenvironment, a bait receptor for an inhibitory molecule was inserted into the NK cell line. Yang et *al.* reported that NK-92 modified to express a dominant negative TGF-β type 2 receptor (DNTβRII) became resistant to TGF-β-mediated inhibitory effects, and thus the transduced NK92 cell line showed more excellent anti-cancer effect than parent NK-29 cell line in the Calu-6 xenograft model (Yang et al. Int. Immunopharmacol. 17: 198-204, 2013). Fourth, the introduction of a chimeric antigen receptor (CAR) to reprogram NK cell lines to recognize a particular antigen is being actively studied. Representatively, anti-CD19-CAR modification allowed NK-92 cells to restore cytotoxicity to previously resistant B-cell leukemia cell lines (Boissel et al., Leuk. Res. 33: 1255-1259, 2009; Romanski et al., J. Cell Mol. Med. 20: 1287-1294, 2016). In addition to these strategies, the introduction of suicide genes into NK cell lines has been proposed to provide a "death switch" that can replace irradiation while simultaneously extending the *in vivo* persistence of injected NK cells. Qu et *al.* recently transferred the inducible caspase 9 (iCasp9) suicide gene to CAR-NK92 cells and demonstrated the elimination of transduced NK cells *in vivo* upon administration of AP20187 (Qu et al., MRS Commun. 9: 433-440, 2019). Multiple genetic engineering strategies are being developed to meet the unmet needs of current NK cell lines, and the integration of these approaches may pave the way for next-generation NK cell line-based immunotherapy.

On the other hand, acute myeloid leukemia (AML) is the most frequent disease among leukemias, accounting for about 70% of acute leukemia, and in the United States, the incidence rate is 4 per 100,000 (Döhner, H. et al., Blood, 115(3): 453-474, 2010), it is reported that more than 13,000 new patients occur every year and about 9,000 people die (SEER Cancer Statistics Review, 1975-2005). Moreover, it is reported that the incidence of AML increases with age, and the annual incidence rate rapidly increases to 15 cases per 100,000 people over 75 years of age and the average age of onset is 67. In addition, 35% of newly diagnosed patients are at least 75 years old (SEER Cancer Statistics Review, 1975-2005).

AML is a cancer in which myeloid cells develop into malignant tumors. Abnormal white blood cells proliferate and accumulate in the bone marrow. Ultimately, if normal blood cells are not produced and proper treatment is not received, the patient will die within weeks or months. Treatment of AML is divided into remission induction chemotherapy to obtain complete remission by reducing leukemia cells to the extent that normal hematopoietic function is restored, and postremission therapy to prevent recurrence after remission. These include consolidation chemotherapy, autologous or allogeneic hematopoietic stem cell transplantation. With initial standard remission-inducing chemotherapy, most (70 - 80%) of young patients (under 60 years of age) can achieve complete remission, but about half of these patients eventually relapse, resulting in recurrence within 5 years. The survival rate is known to be only 40-45%, and patients over 60 years of age have a worse prognosis.

### DISCLOSURE

### TECHNICAL PROBLEM

The present invention is to solve various problems including the above problems, and the object of the present invention is to provide a genetically modified NK cell line transformed to simultaneously express a novel chimeric antigen receptor and a suicide gene effective for the treatment of cancer, particularly acute myeloid leukemia, and a method for treating cancer using the cell line.

### TECHNICAL SOLUTION

According to one aspect of the present invention, there is provided a genetically modified NK cell line expressing a FLT3-specific chimeric antigen receptor by transducing an isolated NK cell line having following characteristic with a polynucleotide encoding an FLT3-specific chimeric antigen receptor:
positive for CD2, CD7, CD11a, CD25, CD28, CD45, CD54, DNAM-1, CD62L and CD56; and
negative for CD1a, CD3, CD4, CD8, CD14, CD20, CD23, CD34, TCRαβ and TCRγδ.

According to another aspect of the present invention, there is provided a cell therapeutic agent for treating cancer containing a therapeutically effective amount of the genetically modified NK cell line as an active ingredient.

In addition, according to another aspect of the present invention, there is provided a cancer treatment kit comprising the genetically modified NK cell line and a suicide inducing agent.

According to another aspect of the present invention, there is provided a method for treating cancer in an individual in need of cancer treatment, comprising administering a therapeutically effective amount of the genetically modified NK cell line and optionally a suicide inducing agent to the individual.

### EFFECT OF THE INVENTION

The genetically modified immune cell that simultaneously expresses a FLT3-specific chimeric antigen receptor protein and a cell suicide gene according to an embodiment of the present invention, and a kit for treating cancer the genetically modified immune cell and a suicide inducing agent are very effective in killing cancer cells. Thus, it, as a new immune cell therapeutic can be usefully used for the treatment of cancer, particularly acute myeloid leukemia, which is a type of existing intractable cancer.

### BRIEF DESCRIPTION OF THE INVENTION

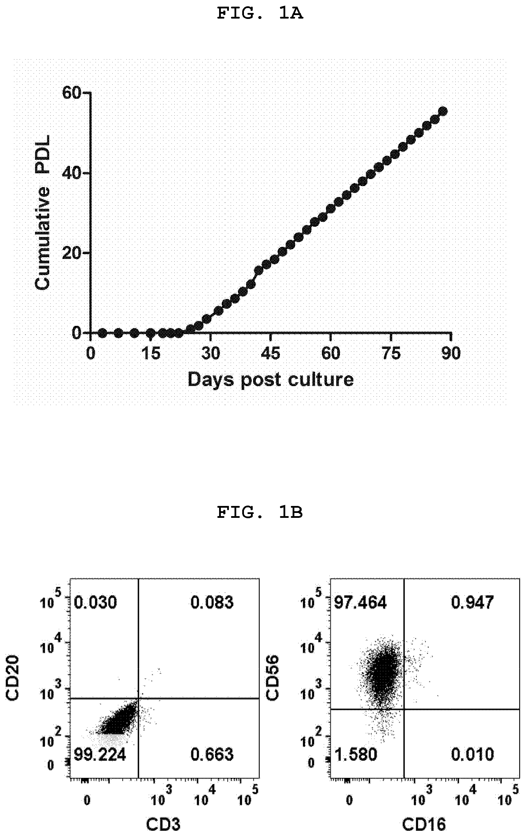

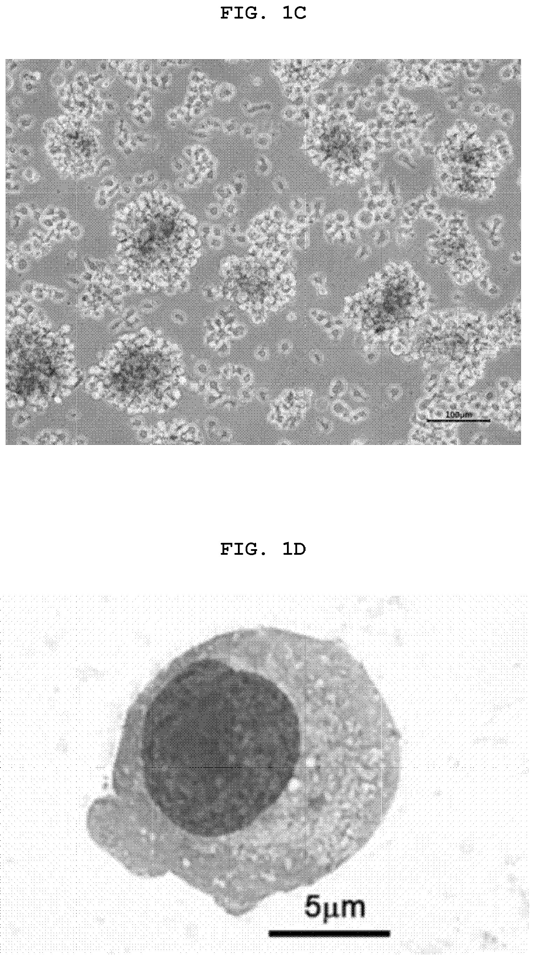

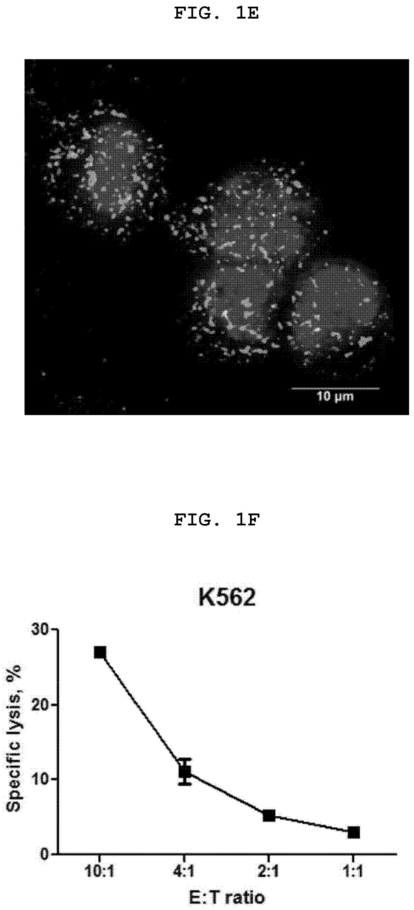

FIG. 1a is a graph showing the degree of cell proliferation of NK101 cell line of the present invention according to passage, and FIG. 1B is a dot graph confirming that NK101 cells are CD3-, CD20-, and CD16-negative and CD56-positive NK cells. FIG. 1C is a photograph of shape of cells identified during culture of NK101 cells using a microscope, FIG. 1D is a photograph of NK101 cells using Wright-Giemsa staining method, FIG. 1E is a photograph confirming that the NK101 cells expressed major cell death factors, Perforin (green) and Granzyme (red) through fluorescent staining method, and FIG. 1F is a graph representing the result of investigating tumor cell killing activity of the NK101 cell line against MHC class I-negative K562 cells through co-cultivating the NK101 cells with the K562 cells.

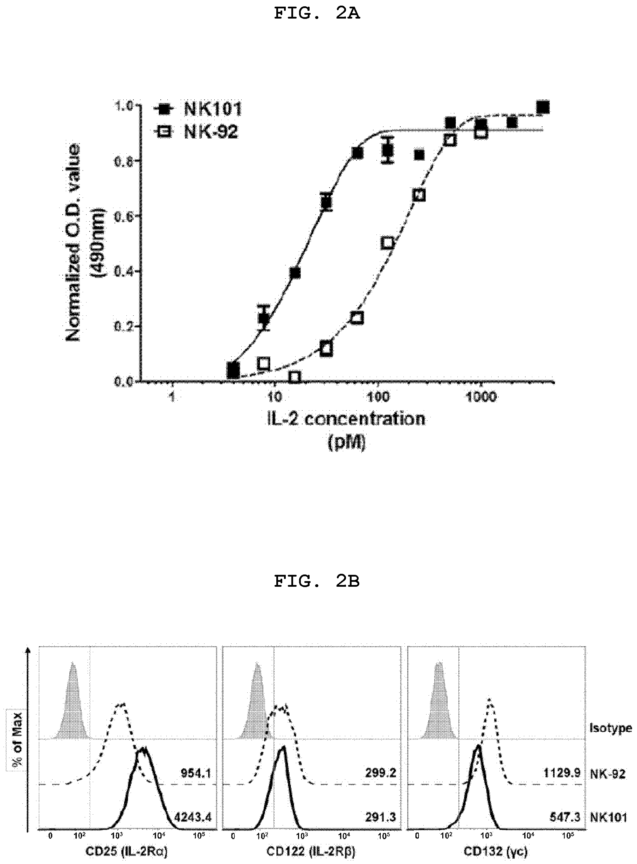

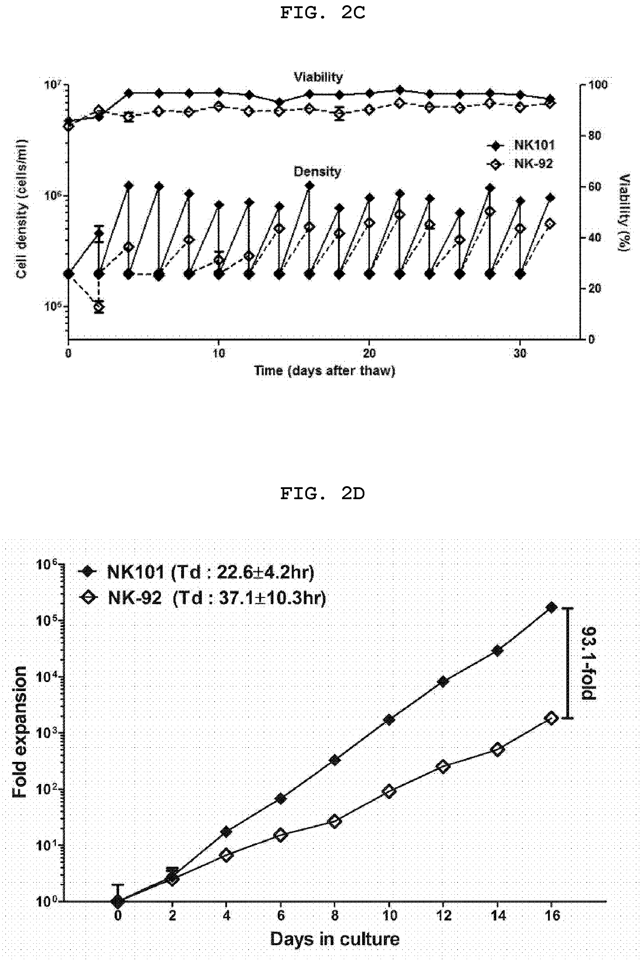

FIG. 2A is a graph representing the result of a comparative analysis of IL-2-dependent cell growth and sensitivity of NK101 and NK-92 through MTS analysis, FIG. 2B is a series of histograms confirming the difference in the expression of IL-2 receptor subunit of NK101 and NK-92 through flow cytometry analyses, and FIG. 2C is a graph representing a comparative analysis of cell growth rate and viability of NK101 and NK-92 from the time of thawing under the same cultivation condition, and FIG. 2D is a graph representing the degree of proliferation and doubling time after culture adaptation.

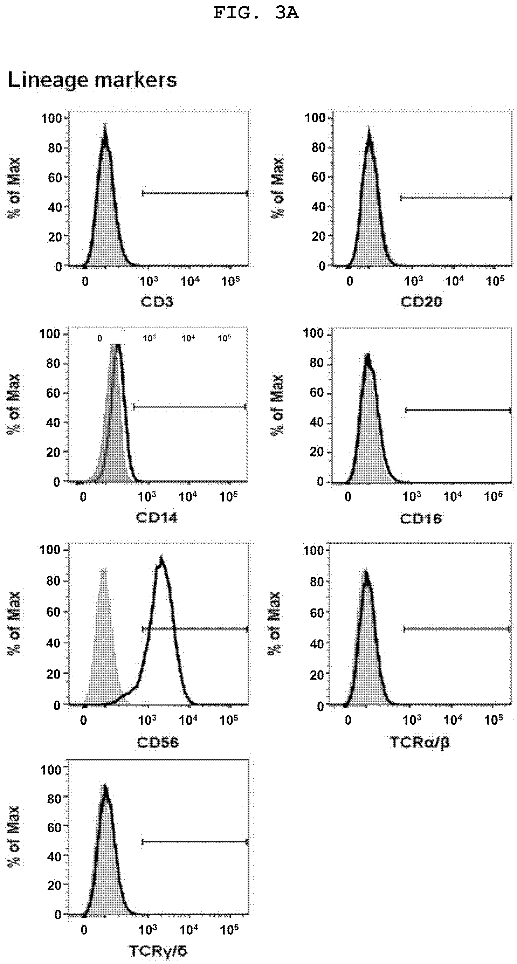

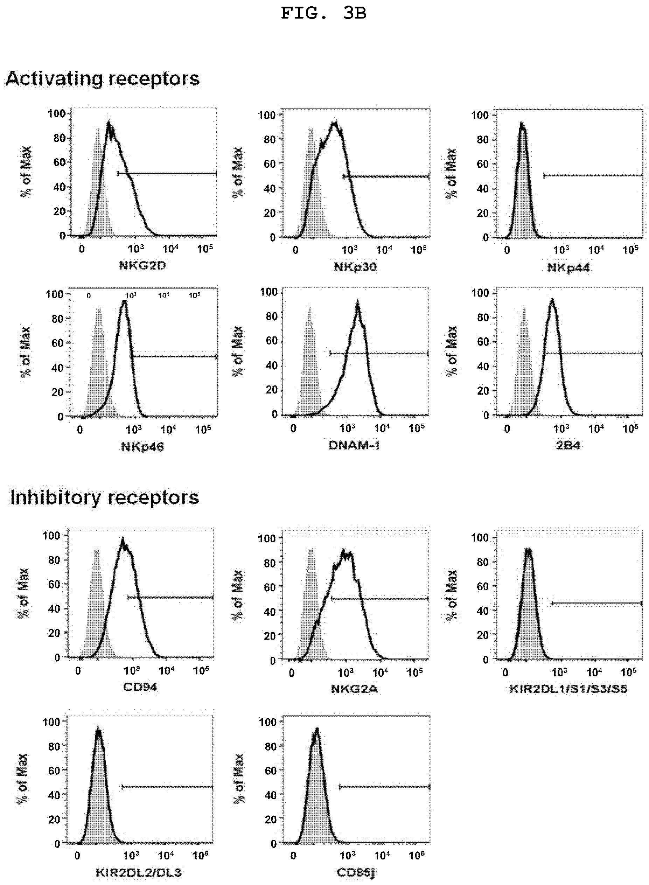

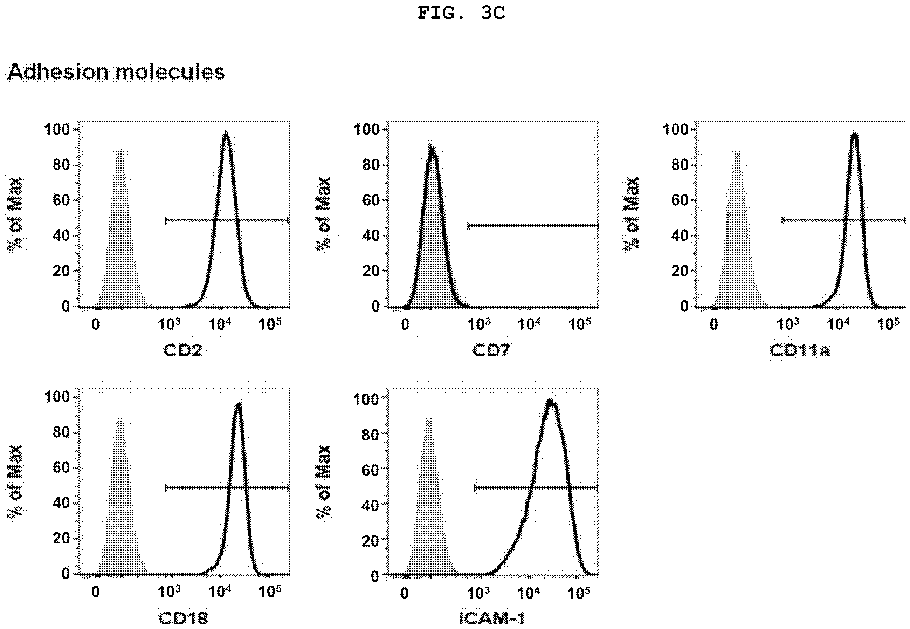

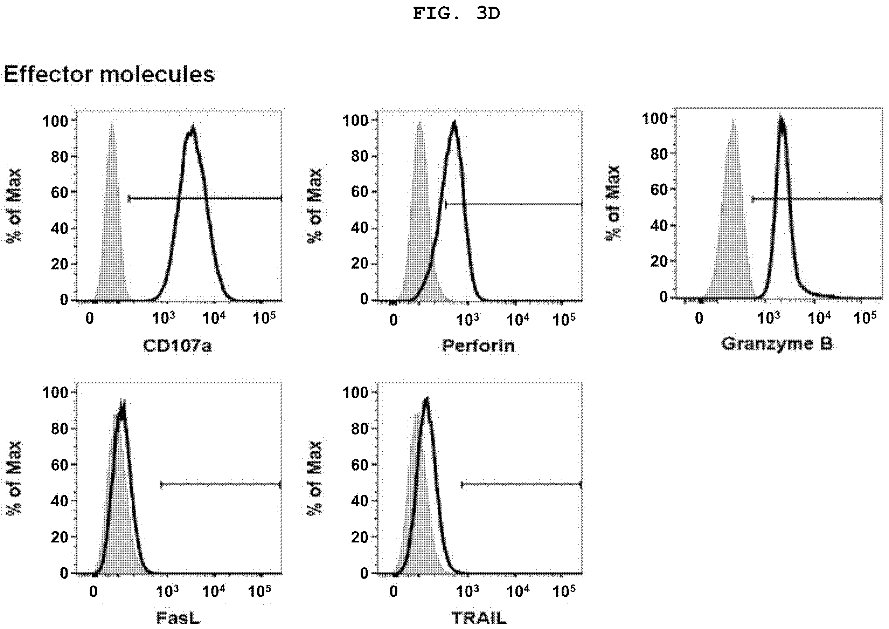

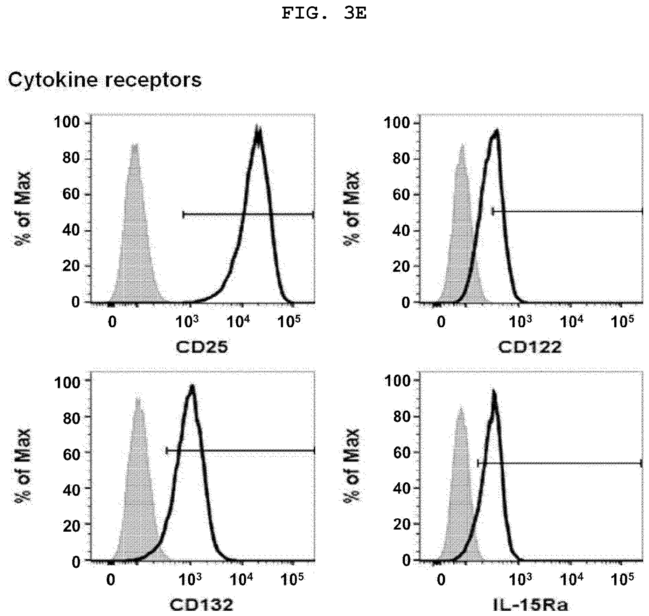

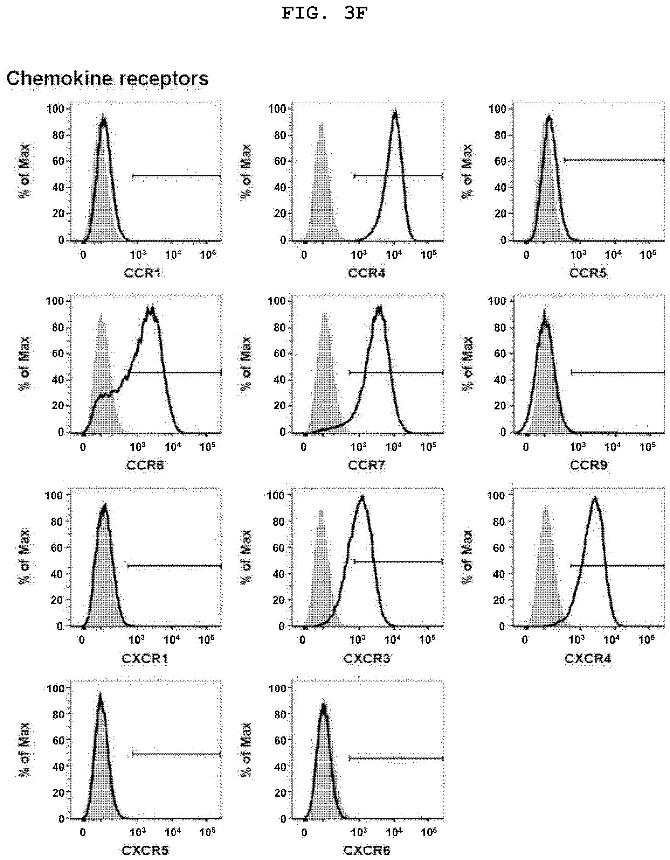

FIG. 3A is a series of histograms showing the results of flow cytometry analyses representing the expression of major lineage or progenitor markers in NK101 cells, FIG. 3B is a series of histograms showing the results of flow cytometry analyses representing the expression of activating receptors and inactivating receptors in NK101 cells, and FIG. 3C is a series of histograms showing the results of flow cytometry analyses representing the expression of cell adhesion factors in NK101 cells, FIG. 3D is a series of histograms showing the results of flow cytometry analyses representing the expression of CD107a, perforin, granzyme, FasL and TRAIL which are involved in NK cell-dependent cytotoxicity in NK101 cells, FIG. 3E is a series of histograms showing the results of flow cytometry analyses representing the expression of cytokine receptors in NK101 cells, and FIG. 3F is a series of histograms showing the results of flow cytometry analyses representing the expression of various C-C chemokine receptors and C-X-C chemokine receptors in NK101 cells.

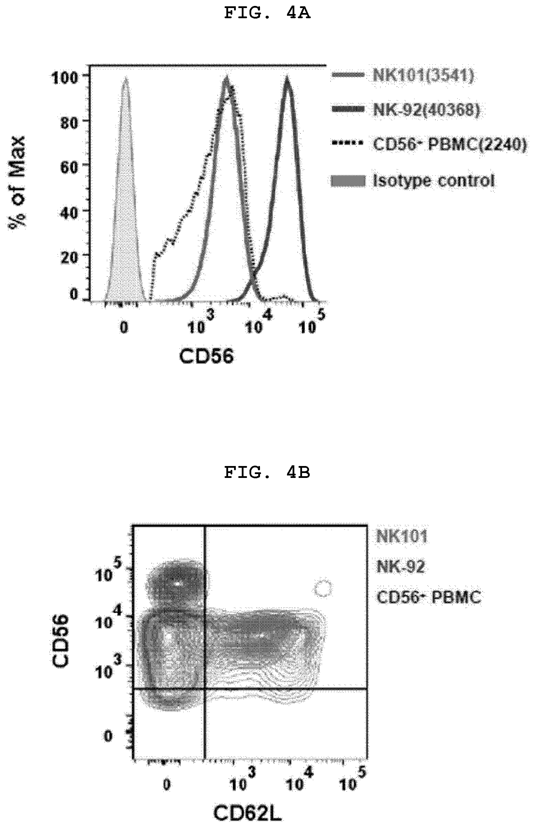

FIG. 4A is a histogram showing the results of flow cytometry analyses representing the expression of CD56 in NK101, NK-92, and primary CD56⁺ peripheral blood NK cells, and FIG. 4B is a two-dimensional contour graph showing the results of flow cytometry analyses representing the expression of CD56 and CD62L in the cells.

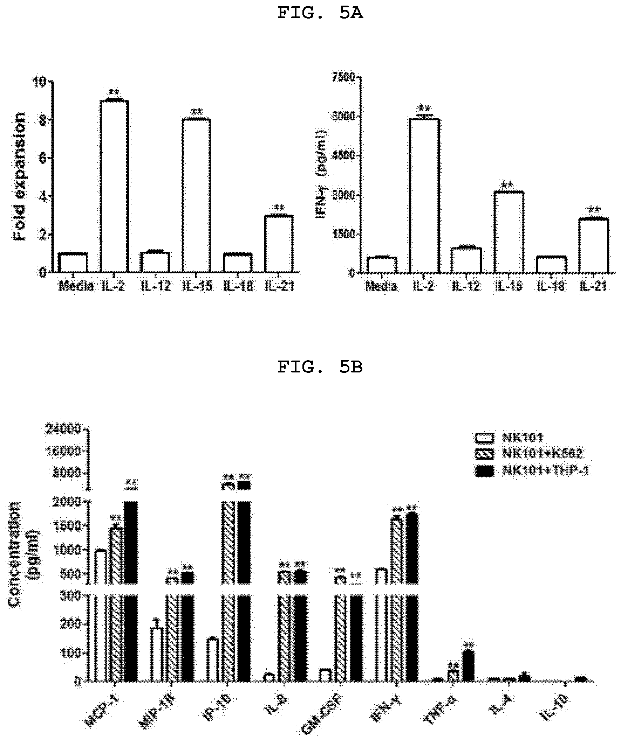

FIG. 5A is a graph (left) showing the results of analysis of proliferation rate of NK101 cells through MTS analysis after treating each indicated cytokine in a culture solution for 3 days and a graph (right) showing the production of IFN-γ, NK-activated cytokine, through ELISA, and FIG. 5B is a graph showing the results of multiplex analysis of the expression of cytokines secreted by NK101 cells after co-cultivating with K562 or THP-1 cancer cells for 24 hours.

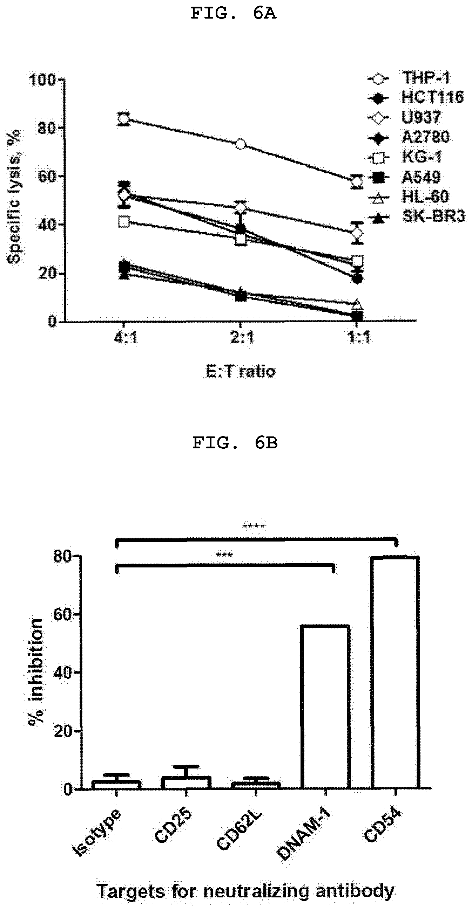

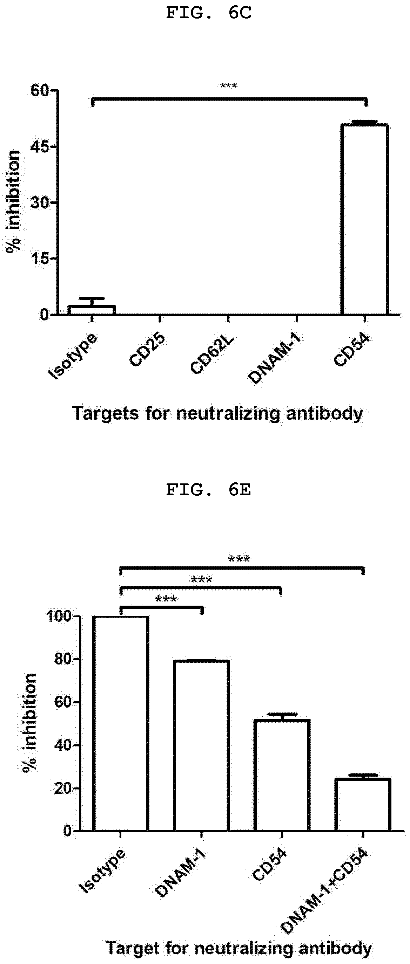

FIG. 6A is a graph showing the apoptotic rate of cancer cells after co-culturing various cancer cell lines with NK101 cells at various effector cells to target cells ratios for 24 hours, and FIG. 6B is a graph representing the apoptotic rate of THP-1, an acute myelogenous leukemia cell line after co-cultivating NK101 cells and the THP-1 cells at an E (effector):T (target) ratio of 4:1, FIG. 6C is a graph showing apoptotic rate of K562, a chronic myeloid leukemia cell line, co-cultivated with NK101 cells at an E:T ratio of 4:1 for 24 hours after treating NK101 cells with each neutralizing antibody against indicated cell surface antigen, FIG. 6D is a graph showing apoptotic rate of Jurkat, an acute lymphocytic leukemia cell line, co-cultivated with NK101 cells at an E:T ratio of 4:1 for 24 hours, after treating the NK101 cells with each neutralizing antibody against indicated cell surface antigen, and FIG. 6E is a graph showing the result of investigating synergistic inhibition by co-neutralization of each surface marker after treating NK101 cells with each neutralizing antibody against DNAM-1, and CD54 or combination of two antibodies against DNAM-1 and CD54.

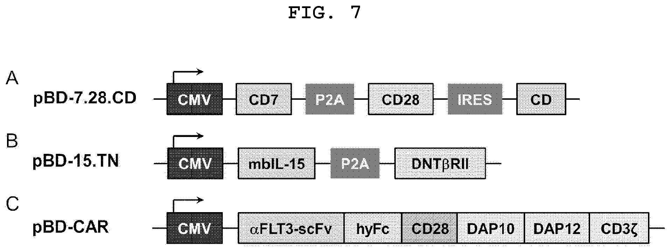

FIG. 7 is a series of vector maps schematically illustrating the structures of lentivirus transfer plasmids (A: pBD-7.28.CD, B: pBD-15.TN, and C: pBD-CAR) used for manufacturing lentiviruses according to an embodiment of the present invention.

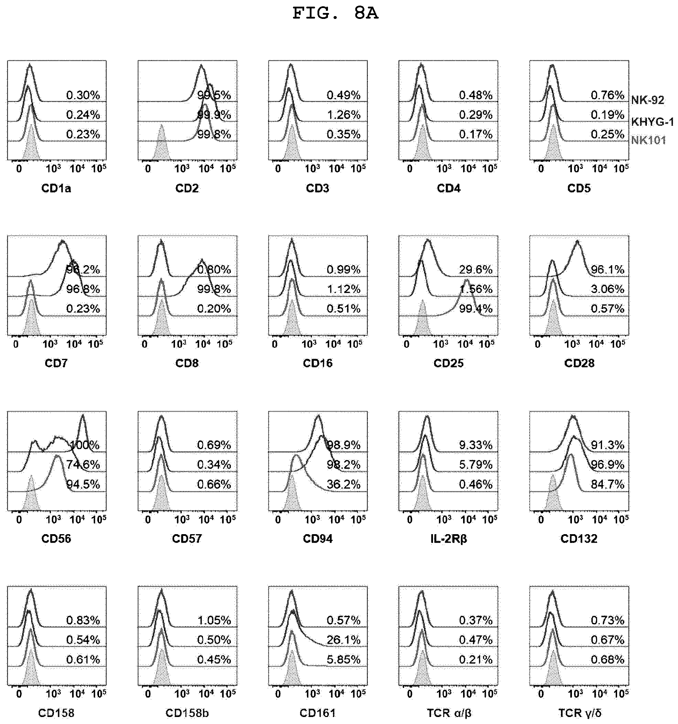

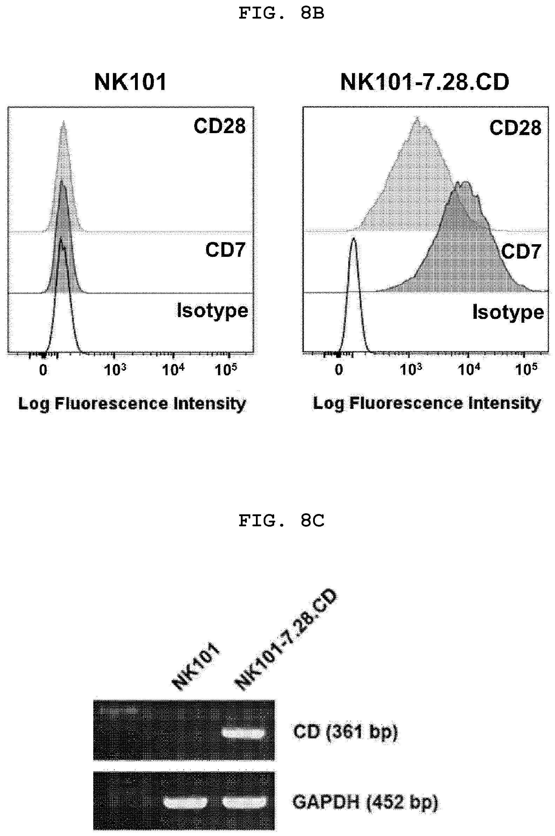

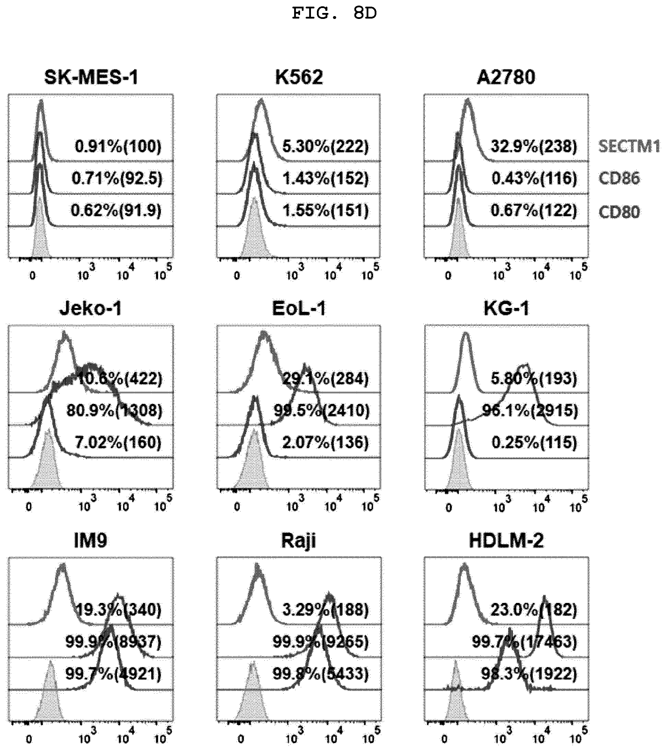

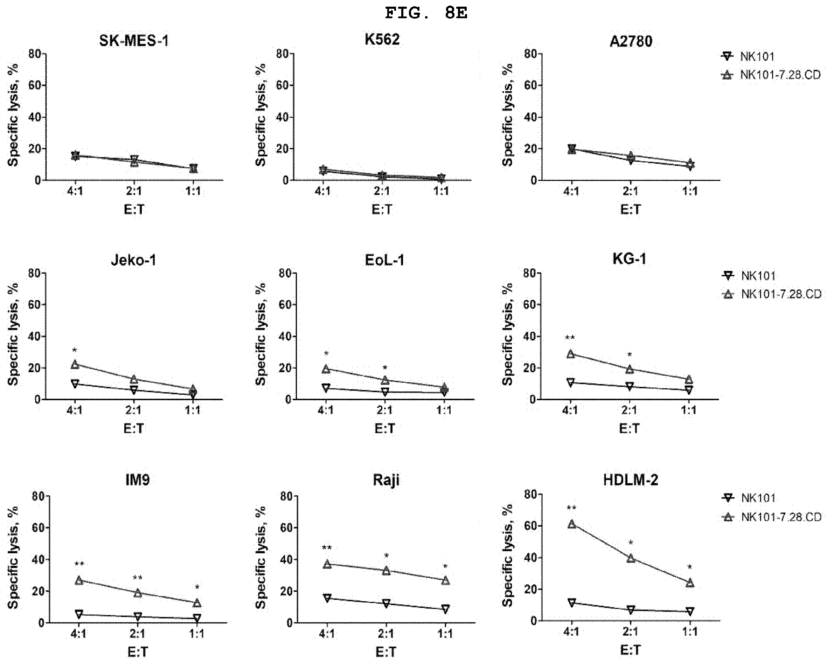

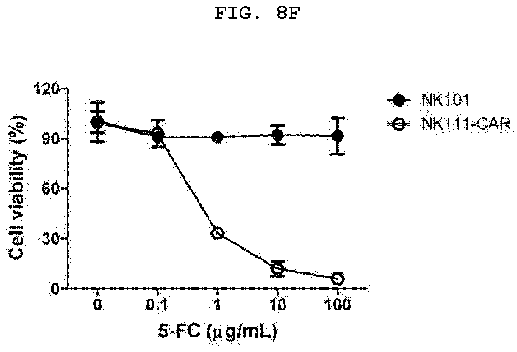

FIG. 8A is a histogram showing the results of flow cytometry analyses representing expression profiles of co-stimulatory factors, CD7 and CD28 in the NK101 cells and previously constructed cell lines KHYG-1, and NK-92, and FIG. 8B is a series of histograms showing the results of flow cytometry analyses representing the expression of CD7, and CD28 in NK101 cells transduced with the gene construct illustrated in FIG. 7B (designated as "NK101-7.28 CD"), FIG. 8C is a photograph showing the result of RT-PCR representing the expression of the CDs in the NK101-7.28.CD cells, FIG. 8D is a series of histograms showing the results of flow cytometry analyses representing the expression of surface CD80 (blue), CD85 (green), and SECTM1 (red) in various cancer cells (SK-MES-1, K562, A2780, Jeko-1, EoL-1, KG-1, IM9, Raji and HDLM-2) and FIG. 8E is a series of graphs representing apoptotic ratio of cancer cells used the above (SK-MES-1, K562, A2780, Jeko-1, EoL-1, KG-1, IM9, Raji and HDLM-2) after co-culturing with NK101 or NK101-7.28.CD cells at E:T ratios of 1:1, 2:1 and 4:1, respectively for 24 hours, through flow cytometry analyses, and FIG. 8F is a graph showing cell growth rate of NK101 or NK101.7.28.CD cells treated with 5-FC at various concentrations for 48 hours determined by MTS analyses.

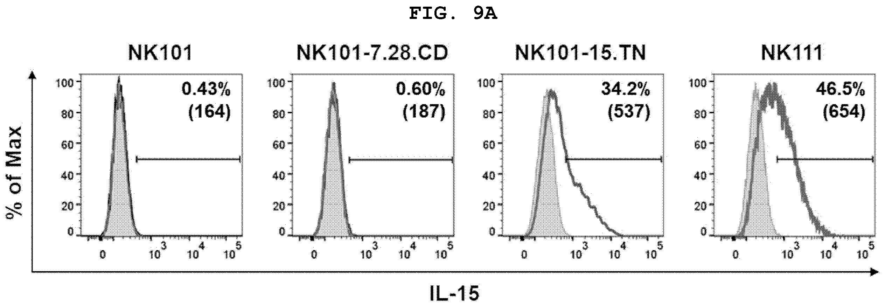

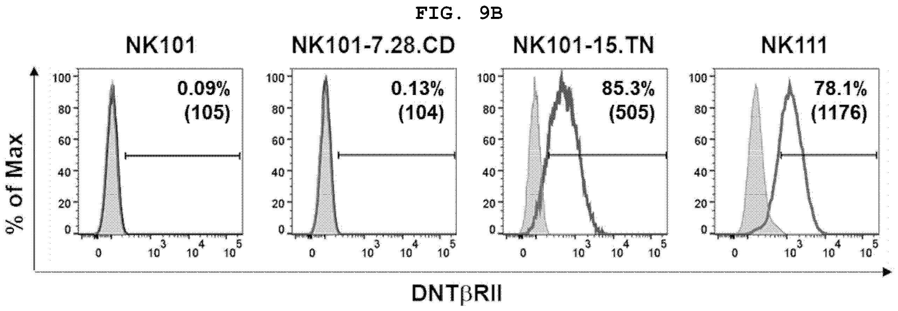

FIG. 9A is a series of histograms showing the results of flow cytometry analyses representing the expression of IL-15 on the surface of NK101 and NK101-15.TN cells, which are prepared by transducing the NK101-7.28.CD cells with the gene construct pBD-15.TN illustrated in FIG. 7, respectively, and FIG. 9B is a series of histograms showing the results of flow cytometry analyses representing the expression of TGFβRIIΔcyto in the NK101-15.TN and NK101 cells.

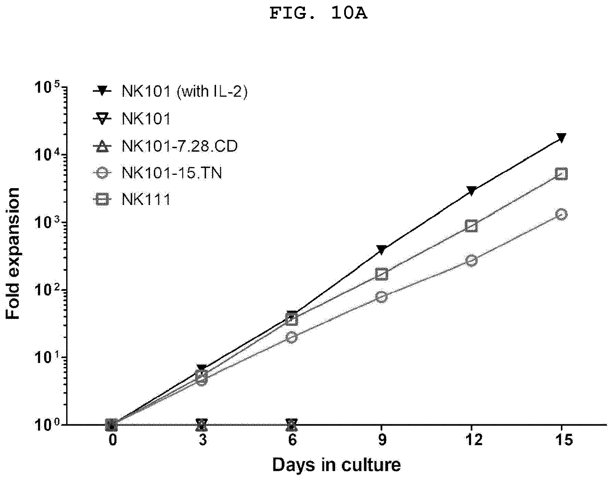

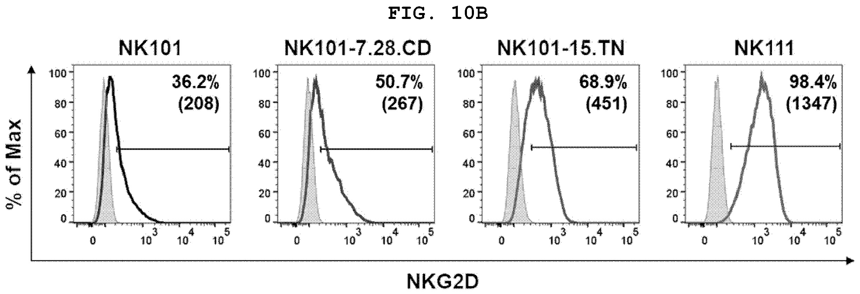

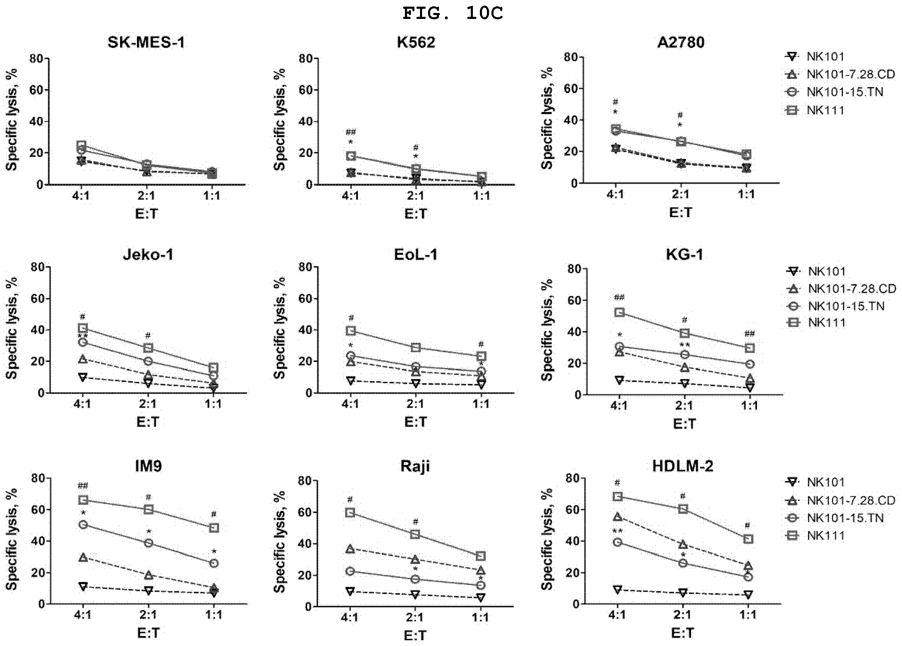

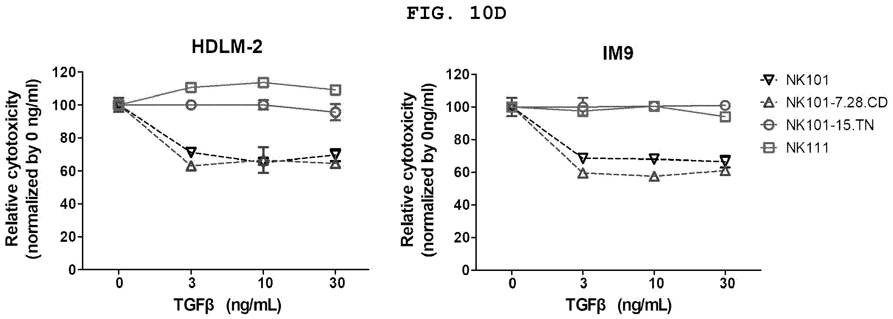

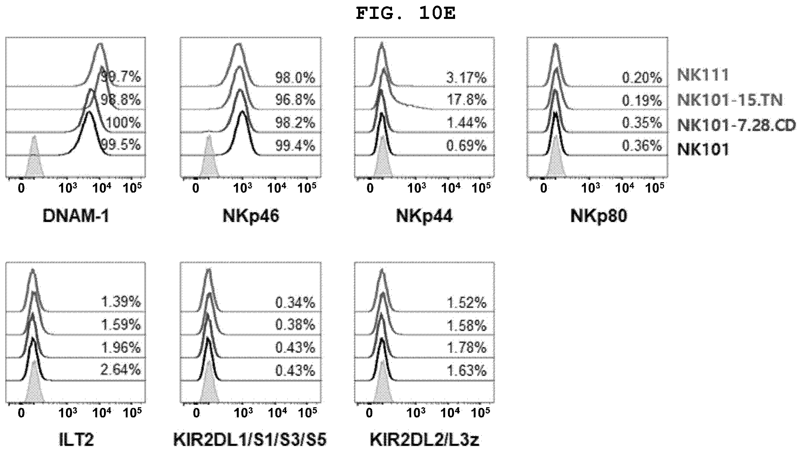

FIG. 10A is a graph showing the population doubling level when IL-2 was not added in the culture medium in the cultivation of NK101, NK101-7.28.CD, NK101-15.TN and NK111 cells, and FIG. 10B is a series of histograms showing the results of flow cytometry analyses representing the expression of NKG2D in the NK101, NK101-7.28.CD, NK101-15.TN and NK111 cells, and FIG. 10C is a series of graphs showing the results of flow cytometry analyses representing cell death ratios of various cancer cells (SK-MES-1, K562, A2780, Jeko-1, EoL-1, KG-1, IM9, Raji and HDLM-2) co-cultivated with NK101, NK101-7.28.CD, NK101-15.TN and NK111 cells, at E:T ratios of 1:1, 2:1, and 4:1, respectively, FIG. 10D is a series of graphs showing the results of cytotoxic analyses representing the change of cancer cell killing activity according to the treatment of TGF-β1 at various concentrations to co-cultivation of HDLM-2 (left) or IM9 (right) cells with NK101, NK101-7.28.CD, NK101-15.TN, and NK111 cells, respectively, and FIG. 10e is a series of histograms showing the results of flow cytometry analyses representing the expression patterns of DNAM-1, NKp46, NKp44, NKp80, ILT2, KIR2DL/S1/S3/S5 and KIR2DL2/L3 in the NK101 (black), NK101-7.28.CD (blue), NK101-15.TN (green) and NK111 (red) cell lines.

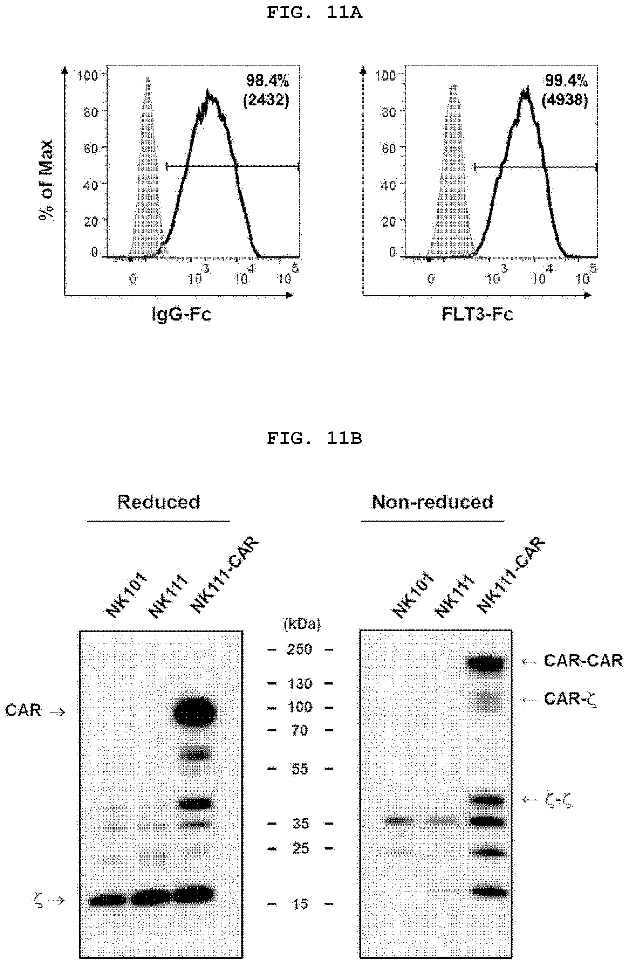

FIG. 11A is a series of histograms showing the result of flow cytometry analysis representing the expression of CAR (left) and the degree of binding of recombinant FLT3-Fc proteins (right) to anti-FLT3 scFv-containing CAR protein on a cell surface of NK cell line in which the pBD-CAR construct illustrated in FIG. 7C is introduced in NK111 (referred to as "NK111-CAR"), according to an embodiment of the present invention.

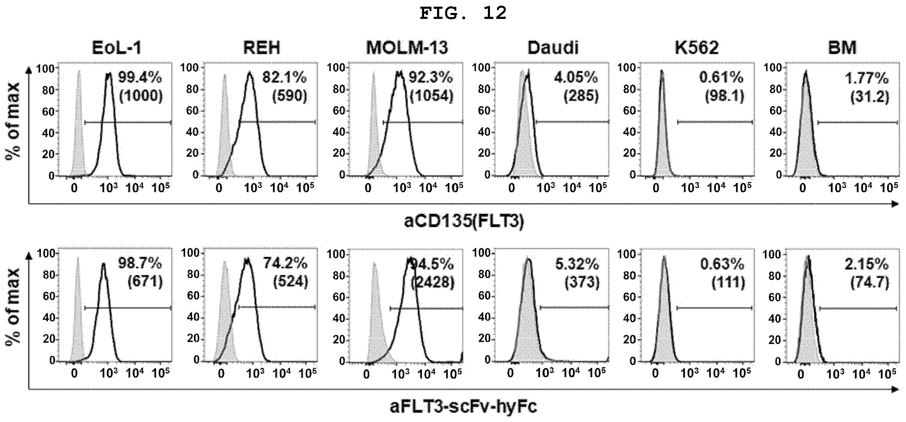

FIG. 12 is a series of histograms showing the results of flow cytometry analyses representing reactivity of a commercial antihuman FLT3 antibody (upper) and the anti-FLT3-scFv-hyFc (lower) according to an embodiment of the present invention to EoL-1, REH, Molm-13, Daudi, K562 cell lines and normal bone marrow-derived peripheral blood cells.

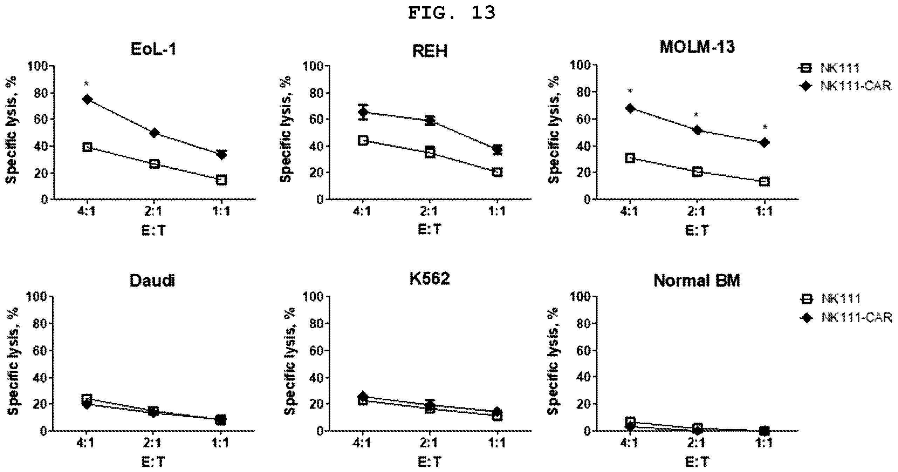

FIG. 13 is a series of graphs showing the results of flow cytometry analyses representing cell apoptotic ratio of various cells (EoL-1, REH, Molm-13, Daudi, K562, and normal bone marrow-derived peripheral blood cells) co-cultivated with NK111 or NK111-CAR cells at E:T ratios of 1:1, 2:1, and 4:1, respectively.

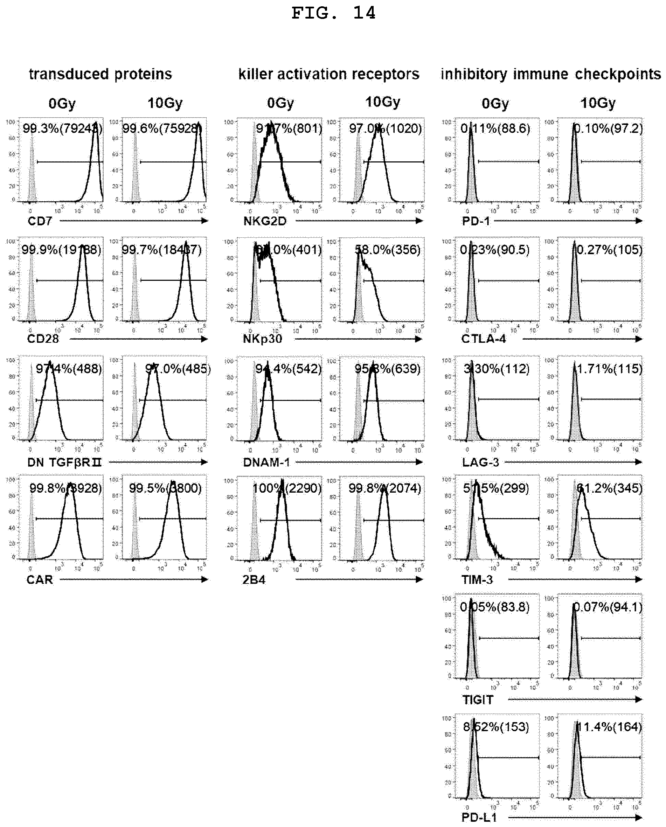

FIG. 14 is a series of histograms showing the results of flow cytometry analyses representing changes in immunophenotype of NK111-CAR cells upon irradiation.

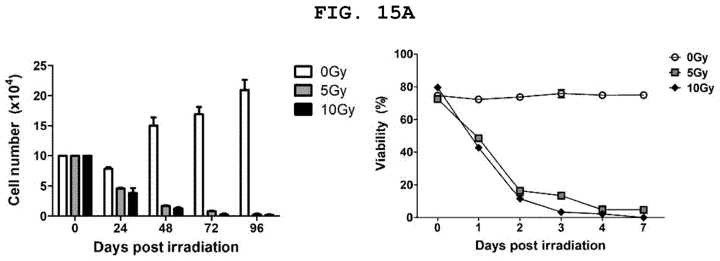

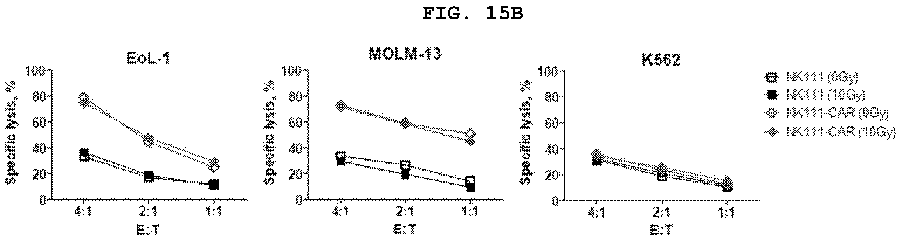

FIG. 15A is a graph showing the number of cells (left) and viability (right) with or without irradiation, and FIG. 15B is a series of graphs showing cancer killing ability of NK111 and NK111-CAR cells after co-cultivating EoL-1, Molm-13 and K562 cancer cells, with the NK111 or NK111-CAR cells at E:T ratios of 1:1, 2:1 and 4:1, respectively in order to analyze apoptotic ratio of the cancer cells with or without irradiation.

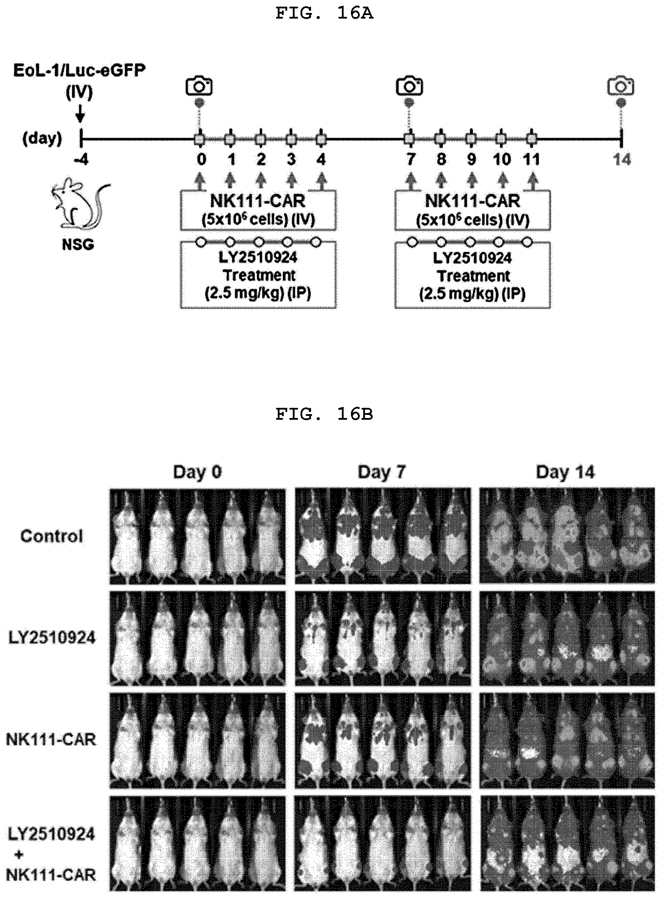

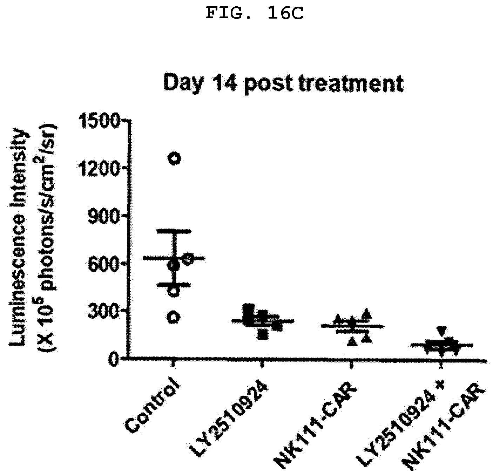

FIG. 16 shows the results of analyzing *in vivo* anticancer efficacy of NK111-CAR cells when NK111-CAR irradiated alone and when LY2510924 which is a CXCR4 antagonist is administered simultaneously to xenograft acute myeloid leukemia model mice,
respectively, FIG. 16A schematically shows the administration schedule of genetically modified NK cell line according to an embodiment of the present invention, FIG 16B is a series of photographs showing the results of whole body imaging representing in vivo distribution of cancer cells after 0, 7, and 14 days from administrating the genetic modified NK cell line according to an embodiment of the present invention and/or LY2510924, FIG. 16C is a graph showing the degree of bioluminescence in the tumor tissues after 14 days from administrating the genetic modified NK cell line and/or LY2510924 to the xenograft model mice.

### BEST MODE

### Definition of terminology:

The term "CAR construct" as used herein stands for "chimeric antigen receptor construct", a synthetic protein consisting of a single chain-based antigen-binding antibody fragment, such as scFv and sdAb, and a co-stimulatory factor-internal signaling domain, it is known that the CAR construct improve anticancer activity of immune cells such as T cells expressing the same by recognizing tumor-specific antigen when the construct is transduced to the immune cells.

The term "scFv" used herein is an abbreviation of "single chain variable fragment", is not an actual antibody fragment, but is known to reserve antigen-binding activity. The scFv is a kind of fusion protein whose size is about 25 a.a. which is prepared by connecting the heavy chain variable region (V_{H}) and the light chain variable region (V_{L}) of an antibody (Glockshuber et al., Biochem. 29(6): 1362-1367, 1990).

The term "genetically modified" as used herein means that host cells (or predecessors/parents) include transduced polynucleotides or vectors according to an embodiment of the present invention in addition to their genomes. In addition, the polynucleotide or vector according to an embodiment of the present invention may be present in genetically modified host cells as independent molecules outside their genome, preferably replicable molecules, or may be stably inserted into the genome of host immune cells.

The term "cell suicide gene" used herein refers to a gene that induces apoptosis of cells expressing the gene by inducing cytotoxicity or triggering apoptotic signaling. In particular, gene expression itself does not trigger apoptosis, but when treating a specific prodrug, prodrug metabolites produced by the apoptotic gene trigger cytotoxicity or apoptotic signaling, leading to apoptosis thereby. These cell suicide genes include the herpes simplex virus thymidine kinase gene (HSV TK) using gancyclovir as a suicide-inducing agent, varicella zoster virus thymidine kinase (VZV TK) using 6-methoxypurine arabinoside as a suicide-inducing agent, cytosine deaminase (CD), uracil phosphoribosyl transferase (UPRT) and a fusion protein of the CD and UPRT using 5-fluorocytosine (5-FC) as a suicide-inducing agent, carboxyl esterase using irinotecan as a suicide-inducing agent, nitroreductase using 5-(aziridin-1-yl)-2,4-dinitrobenzamide (CB1954) as a suicide-inducing agent, carboxypeptidase G2 using 4-((2-chloroethyl)(2-mesiloxyethyl)amino)benzoyl-L-glutamic acid (CMDA) as a suicide-inducing agent, and inducible-caspase 9 (iCas9) using an intermolecular dimerizer as a suicide-inducing agent.

The term "CD::UPRT" used herein is a fusion protein comprising cytosine deaminase (CD) and uracil phosphoribosyl transferase (UPRT) as a cell suicide gene. The CD::UPRT induces cell suicide performing the conversion of 5-fluorocytosine (5-FC) into 5-fluorouracil (5-FU) which is fatal to cells.

The term "mbIL-15" used herein means an IL-15 in the form of binding to the membrane, and is a concept distinguished from secreted IL-15.

The term "TGFβRIIΔcyto" used herein is a variant from which the cytoplasmic domain portion (position corresponding to 192-567 a.a.) of TGFβRII, one of the TGF receptors, has been removed, and is bound to TGF, but cannot transmit intracellular signals.

### Detailed Description of the Invention:

According to one aspect of the present invention, there is provided a genetically modified NK cell line expressing a FLT3-specific chimeric antigen receptor by transducing an isolated NK cell line having following characteristic with a polynucleotide encoding an FLT3-specific chimeric antigen receptor:
positive for CD2, CD7, CD11a, CD25, CD28, CD45, CD54, DNAM-1, CD62L and CD56; and
negative for CD1a, CD3, CD4, CD8, CD14, CD20, CD23, CD34, TCRαβ and TCRγδ.

The genetically modified NK cell line may be further transduced with a cell suicide gene.

In the genetically modified NK cell line, the apoptosis gene may uracil phosphoribosyl transferase (UPRT) gene, herpes simplex virus thymidine kinase (HSV TK) gene, varicella zoster virus thymidine kinase (VZV TK) gene, a cytosine deaminase gene (CD), a gene encoding a fusion protein of the CD and UPRT (CD::UPRT), a carboxyl esterase gene, a nitroreductase gene, a carboxypeptidase G2 gene, or an inducible caspase 9 (iCas9) gene, and the CD::UPRT may consist of an amino acid sequence represented by SEQ ID NO: 20.

The genetically modified NK cell line may be transduced with a polynucleotide encoding membrane-bound IL-15 (mbIL-15).

The genetically modified NK cell line may be transduced with a polynucleotide encoding a cytoplasmic domain-deleted TGFβ receptor II (TGFβRIIΔcyto).

In the genetically modified NK cell line, the isolated NK cell line may be an NK111 cell line in which the CD7-CD28-CD::UPRT construct and the mbIL-15-TGFβRIIΔcyto construct were transduced into the NK101 cell line (KCTC 13305BP)..

In the genetically modified NK cell line, the FLT3-specific chimeric antigen receptor may comprise an scFv that specifically binds to FLT3, a modified Ig Fc region, a CD28 transmembrane domain, a DAP10 intracellular activation domain, a DAP12 intracellular activation domain and CD3ζ cytoplasmic domain of a T cell receptor.

In the genetically modified NK cell line, the scFv that specifically binds to FLT3 may comprises the amino acid sequence represented by SEQ ID NO: 13. The modified Ig Fc domain may comprises the amino acid sequence represented by SEQ ID NO: 1. The CD28 transmembrane domain may comprises the amino acid sequence represented by SEQ ID NO: 3. The DAP10 intracellular activation domain may comprises the amino acid sequence represented by SEQ ID NO: 5. The DAP12 intracellular activation domain may include the amino acid sequence represented by SEQ ID NO: 7. The CD3ζ cytoplasmic domain of the T cell receptor may comprise the amino acid sequence represented by SEQ ID NO: 9. The FLT3-specific chimeric antigen receptor may comprise the amino acid sequence represented by SEQ ID NO: 11, and the polynucleotide encoding the FLT3-specific chimeric antigen receptor may comprise the nucleic acid sequence represented by SEQ ID NO: 12.

As used herein, the term "DAP10" is a transmembrane signal adapter that forms an immune receptor complex, and refers to a hematopoietic cell signal transducer encoded by a hematopoietic cell signal transducer (HCST) gene, and it is known to play an important role in the survival and proliferation of cells by the activation of NK cells and T cell reaction.

As used herein, the term "DAP12" is a 12 kDa transmembrane protein, has high homology with DAP10, and is recognized as an important signaling receptor in NK cells.

In the genetically modified NK cell line, the cell suicide gene may be uracil phosphoribosyl transferase (UPRT) gene, herpes simplex virus thymidine kinase (HSV TK), varicella zoster virus thymidine kinase gene (VZV TK), a cytosine deaminase gene (CD), a gene encoding a fusion protein of the CD and UPRT (CD::UPRT), a carboxyl esterase gene, a nitroreductase gene, a carboxypeptidase G2 gene, or an inducible caspase 9 gene (iCas9). In addition, the CD::UPRT may comprise the amino acid sequence represented by SEQ ID NO: 20.

In the genetically modified NK cell line, the mbIL-15 may comprise the amino acid sequence represented by SEQ ID NO: 23.

In the genetically modified NK cell line, the TGFβRIIΔcyto may comprise the amino acid sequence represented by SEQ ID NO: 23.

As used herein, the term "chimeric antigen receptor (CAR)" refers to a type of fusion protein prepared by fusing an antigen-binding portion (variable region) of a monoclonal antibody to an intracellular signaling site derived from a lymphocyte activation receptor. When these chimeric antigen receptors are expressed in human T cells, they can induce potent cellular mechanisms of action without limitation of the major histocompatibility complex (MHC). The potential of this approach has been reported in clinical studies in which CAR-expressing T cells were injected into pancreatic cancer patients. Sustained efficacy and remarkable objective responses have been observed in resistant patients, and the use of CAR technology is expected to be used more extensively in cancer treatment. Currently, third-generation CAR molecules after the first- and second-generations CAR molecules have been reported.

More specifically, the FLT3-specific chimeric antigen receptor, CD7-CD28-CD::UPRT construct and mbIL-15-TGFβRIIΔcyto construct may be expressed in the form of a fusion protein or may be co-expressed by transfecting host immune cells with a single gene construct comprising all the components, or co-expressed by co-transfecting host immune cells with separate gene construct containing the components, respectively. When the polynucleotide encoding the FLT3-specific chimeric antigen receptor, the CD7-CD28-CD::UPRT construct and the mbIL-15-TGFβRIIΔcyto construct are cloned as a single gene construct, each may be operably linked to separate promoters or polynucleotides encoding the FLT3-specific chimeric antigen receptor, the CD7-CD28-CD::UPRT construct and the mbIL-15-TGFβRIIΔcyto construct, respectively are linked under the control of a single promoter but each polynucleotide may be linked with internal ribosome entry site (IRES) and thus may be expressed polycistronically.

As used herein, "operably linked to" means that a specific polynucleotide is linked to another polynucleotide so that it can exert its function. That is, when a polynucleotide encoding a particular protein is operably linked to a promoter, it means that it is transcribed into mRNA by the action of the promoter and linked so as to be translated into the protein, and the polynucleotide encoding a particular protein is operably linked to a polynucleotide encoding another protein means that the particular protein may be expressed in the form of a fusion protein with another protein.

The gene construct may be cloned into an expression vector to transfect host immune cells, and this vector may include various regulatory elements enabling expression of the cloned gene therein, including a promoter. Such regulatory elements are well known to those skilled in the art. As mentioned above, these usually include elements responsible for transcription initiation and, optionally, poly-A signals responsible for transcription termination and stabilization of transcripts. Additional regulatory elements may include, in addition to transcriptional regulatory elements, translation enhancers and/or naturally-combined or heterologous promoter regions. Possible regulatory elements that enable expression in the mammal host cells, for example, may include CMV-HSV thymidine kinase promoter, SV40 promoter, RSV (Rous sarcoma virus) promoter, human elongation factor 1α-promoter (hEF1α protomer), glucocorticoid-inducible MMTV (Moloni mouse tumor virus) promoters, metallothionein-inducible promoter or tetracycline-inducible promoter, or enhancers such as a CMV enhancer or an SV40-enhancer. For the expression in neurons, it is contemplated that neurofilament promoter, PGDF promoter, NSE promoter, PrP promoter or thy-1 promoter may be used. Such promoters are known in the art and are described in Charron et al. (J. Biol. Chem. 270: 25739-25745, 1995). In addition to the regulatory elements capable of initiating transcription, the regulatory elements may include transcription termination signals such as an SV40-poly-A site or a TK-poly-A site downstream of the polynucleotide according to an embodiment of the present invention. Suitable expression vectors being used herein are known in the art, for example, Okayama-Berg cDNA expression vectors pcDV1 (Parmacia), pRc/CMV, pcDNA1, pcDNA3 (In-vitrogene), pSPORT1 (GIBCO BRL), pX (Pagano, Science 255: 1144-1147, 1992), yeast two-hybrid vectors such as pEG202 and dpJG4-5 (Gyuris, Cell 75: 791-803, 2005).

According to another aspect of the present invention, there is provided a cell therapeutic agent for treating cancer containing a therapeutically effective amount of the genetically modified NK cell line as an active ingredient.

The cell therapeutic agent for treating cancer may further include at least one or more other anticancer agents. The anticancer agent may be an alkylating agent, antimetabolites, anti-microtubule agents, topoisomerase inhibitors, cytotoxic agents, cell proliferation/migration and survival signaling pathway inhibitors, cell suicide inducing agents, or histone deacetylase (HDAC) inhibitors.

In the cell therapeutic agent for cancer treatment, the alkylating agent may be nitrogen mustard, nitrosourea, tetrazine, aziridine, or cisplatin, and the antimetabolites are methotrexat, pemetrexed, fluorouracil, capecitabine, cytarabine, gemcitabine, decitabine, azacitidine, fludarabine, nelarabine, cladribine, clofarabine, or pentostatin. The anti-microtubule agent may be vincristine, vinblastine, vinorelbine, vindesine, vinflunine, paclitaxel, or pdophyllotoxin. The topoisomerase inhibitor may be irinotecan, doxorubicinside, toptecan, mitoxantrone, novobiocin, merbarone, aclarubicin or teniposide. The cytotoxic agent may be anthracycline, bleomycin, mitomycin C, mitoxantrone or actinomycin. The cell proliferation/migration and survival signaling pathway inhibitor may be a PI3K/AKT/mTOR inhibitor, or a CXCR4 peptide antagonist. In this case, the CXCR4 peptide antagonist may be Plerixafor, BL-8040 or LY2510924.

The cancer capable of being treated by the cell therapeutic agent for treating cancer of the present invention may be either a hematological cancer or a solid cancer as long as it is a cancer in which FLT3 is overexpressed, and the hematological cancer may be acute and chronic leukemia, lymphoma, multiple myelopathy or myelodysplastic syndrome. The solid cancer may be liver cancer, lung cancer, pancreatic cancer, breast cancer, ovarian cancer, endometrial cancer, cervical cancer, gallbladder cancer, stomach cancer, biliary tract cancer, colorectal cancer, head and neck cancer, esophageal cancer, thyroid cancer, brain tumor, malignant melanoma, prostate cancer, testicular cancer, tongue cancer, or bone marrow cancer.

The cell therapeutic agent for treating cancer may be a kind of pharmaceutical composition, and may include at least one pharmaceutically acceptable carrier, additive or excipient necessary for the formulation of the pharmaceutical composition.

Meanwhile, the dosage of the cell therapeutic agent for treating cancer according to an embodiment of the present invention may be 10⁷ to 10¹¹ cells, but may be adjusted according to the patient's sex, age, disease progression, and treatment purpose. Generally, such an amount will be sufficient to carry out localization in target cancer cells overexpressing a cancer antigen, and kill the cancer cell, e.g., by phagocytosis or cytolysis.

The cell therapeutic agent may further include a pharmaceutically acceptable carrier, diluent, or excipient in addition to the carrier.

In addition, the "pharmaceutically acceptable" refers to a composition that is physiologically acceptable and does not normally cause allergic reactions such as gastrointestinal disorders, dizziness, or similar reactions when administered to humans. The composition according to an embodiment of the present invention may be formulated in a suitable form together with a commonly used pharmaceutically acceptable carrier. Pharmaceutically acceptable carriers include, for example, carriers for parenteral administration such as water, suitable oils, saline, aqueous glucose and glycol, and the like, and may further include stabilizers and preservatives. Suitable stabilizers include antioxidants such as sodium bisulfite, sodium sulfite or ascorbic acid. Suitable preservatives include benzalkonium chloride, methyl- or propyl-paraben and chlorobutanol. In addition, the cell therapy agent according to the present invention is a suspending agent, solubilizing agent, stabilizer, isotonic agent, preservative, adsorption inhibitor, surfactant, diluent, excipient, pH adjuster, analgesic agent, buffer, if necessary depending on the administration method or formulation, antioxidants and the like may be included as appropriate. Pharmaceutically acceptable carriers and agents suitable for the present invention, including those exemplified above, are described in detail in Remington's Pharmaceutical Sciences, latest edition.

In addition, the cell therapeutic agent according to an embodiment of the present invention may be formulated using a method known in the art to enable rapid, sustained or delayed release of the active ingredient when administered to a mammal. Formulations include powders, granules, tablets, emulsions, syrups, aerosols, soft or hard gelatin capsules, sterile injectable solutions, and sterile powder forms.

The composition according to an embodiment of the present invention may be administered by various routes, for example, oral, parenteral, for example, suppository, transdermal, intravenous, intraperitoneal, intramuscular, intralesional, intracranial, intranasal, or intrathecal, and it can also be administered using implanted devices for sustained release or continuous or repeated release. The number of administrations may be administered once or divided into several times a day within a desired range, and the administration period is not particularly limited.

The dosage of the pharmaceutical composition to a patient depends on many factors including the patient's height, body surface area, age, the particular compound being administered, sex, time and route of administration, general health of patients, and other drugs being administered simultaneously. The pharmaceutically active protein may be present in an amount of 1 ng - 10 mg/body weight (kg) per administration, but may be administered below or above the exemplary range in consideration of the above factors, and if the administration method is continuous infusion, it can be administered within the range of 1 µg - 10 mg per 1 kg of body weight per minute.

In addition, according to another aspect of the present invention, there is provided a kit for treating cancer comprising the genetically modified NK cell line and a suicide inducing agent.

The kit for treating cancer may further include at least one or more other anticancer agents. The anticancer agent may be an alkylating agent, antimetabolites, anti-microtubule agents, topoisomerase inhibitors, cytotoxic agents, cell proliferation/migration and survival signaling pathway inhibitors, apoptosis inducers, or histone deacetylase (HDAC) inhibitors. Specific anticancer agents belonging to the above-described subcategory are as described above.

The kit for treating cancer according to an embodiment of the present invention may include the genetically modified immune cell and a suicide inducing agent, but these two components are not provided in a mixed form, but are packaged and provided separately, and these two components are administered at the same time by the same or a different route. Alternately, it may be administered sequentially or at a regular interval, according to a doctor's prescription, thus it is distinguished from a conventional pharmaceutical composition. For example, according to one use aspect of the kit of the present invention, firstly the genetically modified immune cells are administrated at an appropriate time point, and then at 1st day, 2nd day, 3rd day, 4^{th} day, 5th day, 6^{th} day, 7^{th} day, 10^{th} day, after 2^{nd} week, after 15^{th} day, after 20^{th} day, after 3^{rd} week, after 25^{th} day, after 4^{th} week, or after 30^{th} day from the administration of the immune cells, the cell suicide inducing agent may be administrated and the suicide inducing agent may be multiply administered at intervals of two, three, four, five, six days, or one week from the first administration.

In the kit of the present invention, the suicide inducing agent may be ganciclovir or 6-methoxypurine arabinoside when the cell suicide gene is HSV TK or VZV TK, respectively, and the suicide inducing agent may be 5-fluorocytosine (5-FC) when the cell suicide gene is cytosine deaminase (CD) gene, uracil phosphoribosyl transferase (UPRT) gene or a gene encoding a fusion protein of the CD and UPRT (CD::UPRT), and the suicide inducing agent may be irinotecan (CPT-11) when the cell suicide gene is carboxyl esterase, and the suicide inducing agent may be 5-(aziridin-1-yl)-2,4-dinitrobenzamide (CB1954) when the cell suicide gene is nitroreductase, and the suicide inducing agent may be 4-((2-chloroethyl)(2-mesiloxyethyl)amino)benzoyl-L-glutamic acid (CMDA) when the cell suicide gene is carboxypeptidase G2, and the suicide inducing agent may be iCas9 dimerizer when cell suicide gene is iCas9.

According to another aspect of the present invention, there is provided a method for treating cancer in an individual in need of cancer treatment, comprising administering a therapeutically effective amount of the genetically modified NK cell line and optionally a suicide inducing agent to the individual.

The method may further comprise performing one or more anticancer therapies selected from stem cell transplantation, radiation therapy, surgical resection, chemotherapy, immunotherapy, targeted therapeutics, or a combination thereof.

The genetically modified NK cell line and the suicide inducing agent may be administered simultaneously, but as described above, they may be administered at an appropriate administration interval for optimal effect, and the administration interval may be adjusted to maximize therapeutic efficacy.

Cancers capable being treated by the method according to an embodiment of the present invention may be either hematologic cancers or solid cancers as long as FLT3 is overexpressed in the cancer cells, and the hematologic cancers may be acute and chronic leukemia, lymphoma, multiple myelopathy or myelodysplastic syndrome. In addition, the solid cancer may be liver cancer, lung cancer, pancreatic cancer, breast cancer, ovarian cancer, endometrial cancer, cervical cancer, gallbladder cancer, stomach cancer, biliary tract cancer, colorectal cancer, head and neck cancer, esophageal cancer, thyroid cancer, brain tumor, malignant melanoma, prostate cancer, or tongue cancer, or bone marrow cancer.

Hereinafter, the present invention will be described in more detail with reference to the accompanying drawings.

The present inventors isolated a novel NK cell line having the properties described in Table 1 from the cancer tissue of a patient who suffering from NK lymphoma, and as a result of examining various properties thereof, as shown in FIGs 1 to 6, it was confirmed that the NK cell line exhibits IL-2-dependents proliferative property and it is a multifunctional NK cell line having both cancer cell killing activity and immunomodulatory ability. In particular, compared to NK-92, the only NK cell line currently undergoing clinical trials, it has been confirmed that it is a cell line capable of being produced on a commercial scale because it has significantly higher proliferative capacity. In this reason, it was deposited at the Korean Collection for Type Culture (KCTC) within the Korea Research Institute of Bioscience and Biotechnology on August 7, 2017, and was given an accession number of KCTC 13305BP on August 24, 2017 and designated as "NK101". However, as a result of gene expression analysis of the NK101 cell line, it was confirmed that the IL-2 receptor CD25 was highly expressed and had a CD56^{dim}CD62L⁺ phenotype. By confirming that it does not express CD7 and CD28 which are major NK cell activating factors affecting on anti-cancer activity of NK cells, it was expected that the anticancer activity itself would not be higher than that of the NK cell line available in the prior art.

Accordingly, the present inventors tried to prepare a genetically modified NK cell line based on the NK101 cell line in order to enhance the anticancer activity of NK101 cell line according to an embodiment of the present invention. FIG 7A is a schematic diagram schematically illustrating the structure of the gene construct pBD-7.28.CD used for the preparation of the genetically modified NK cell line NK101-7.28.CD according to an embodiment of the present invention to achieve the above object; FIG. 7B is a schematic diagram schematically illustrating the structure of the gene construct pBD-15.TN used for the preparation of the genetically modified NK cell lines NK101-15.TN and NK111 according to an embodiment of the present invention.

In the present invention, in order to complement the above-described factors, the present inventors introduced a co-stimulatory receptor of NK cells, a cell suicide gene, a membrane bound cytokine, a TGFβRII variant protein in the NK101 cells (Accession No. KCTC 13305BP) and constructed a genetically modified NK cell line (NK111) whose anticancer effect is enhanced and cytokine supplement-independent proliferation is possible thereby (FIGs. 9A and 9B).

FIG. 8A shows the results of comparative analyses of expression profiles of cell surface markers in the genetically modified NK cell line (NK101-7.28.CD) according to an embodiment of the present invention, NK101 cell line which is a parent cell line of the NK101-7.28.CD, KHYG-1 and NK-92 which are previously constructed NK cell lines, and FIG. 8B is a series of histograms showing the results of flow cytometry analyses representing the expression of CD7, and CD28 in NK101 cells transduced with the gene construct illustrated in FIG. 7B (designated as "NK101-7.28 CD"). As shown in FIGs. 8A and 8B, KHYG-1 and NK-92, which are known to have high cancer cell killing activity among previously constructed NK cell lines, have a characteristic of expressing CD7 in common, and the NK-92 cell line also express CD28 in addition to CD7, whereas it was confirmed that both co-stimulatory factors were not expressed at all in NK101. Accordingly, the present inventors tried to evaluate whether cancer cell killing activity is enhanced through the introduction of CD7 and CD28 into NK101 cells.

FIG. 8D is a series of histograms showing the results of flow cytometry analyses representing the expression of surface CD80 (blue), CD85 (green), and SECTM1 (red) in various cancer cells (SK-MES-1, K562, A2780, Jeko-1, EoL-1, KG-1, IM9, Raji and HDLM-2) FIG. 8E shows cancer cell killing activity of the genetically modified NK cell line (NK101-7.28.CD), NK101 which is a parent cell line of the NK101-7.28.CD) against various cancer cells (SK-MES-1, K562, A2780, Jeko-1, EoL-1, KG-1, IM9, Raji and HDLM-2), which is a series of graphs representing cell death ration of cancer cells used the above (SK-MES-1, K562, A2780, Jeko-1, EoL-1, KG-1, IM9, Raji and HDLM-2) after co-culturing with NK101 or NK101-7.28.CD cells at E:T ratios of 1:1, 2:1 and 4:1, respectively for 24 hours, through flow cytometry analyses. As shown in FIGs. 8E and 8F, the genetically modified NK cell line NK101-7.28.CD according to an embodiment of the present invention showed excellent cancer cell killing activity in various cancer cell lines compared to NK101, suggesting that the cancer cell killing activity of NK101 cells can be improved by the introduction of CD7 and CD28. In addition, it was confirmed that safety can be secured by inducing the removal of NK101-7.28.CD cells through 5-FC treatment.

FIG. 9 shows the construction process of the NK111 cell line, which is a genetically modified NK cell line, according to another embodiment of the present invention. FIG. 9A is a series of histograms showing the results of flow cytometry analyses representing the expression of IL-15 on the surface of NK101 and NK101-15.TN cells, which are prepared by transducing the NK101-7.28.CD cells with the gene construct pBD-15.TN illustrated in FIG. 7, respectively, and FIG. 9B is a series of histograms showing the results of flow cytometry analyses representing the expression of TGFβRIIΔcyto in the NK101-15.TN and NK101 cells. As shown in FIG. 8E, the introduction of CD7 and CD28 had a moderate effect on enhancing the cancer cell killing activity of NK101 cells. Accordingly, the present inventors tried to transduce NK101-7.28.CD cells with membrane bound IL-15, a representative NK cell activating factor, into NK101-7.28.CD cells in order to maximize the cancer cell killing activity of NK101-7.28.CD cells, and in order to induce resistance to TGF-β, which is an immunosuppressive factor, TGFβRIIΔcyto (dominant negative TOPPβKII DNTβRII) with a deleted cytoplasmic domain was overexpressed to act as a decoy receptor. As a result, as shown in FIGs. 9A and 9B, it was confirmed that membrane-bound IL-15 and dominant negative TGFβRII, which were not expressed in the parental cell line NK101-7.28.CD, were expressed in the NK111 cell line according to an embodiment of the present invention.

FIGs. 10A to 10E show the results of comparative analysis of anticancer activity and safety of NK111, a genetically modified NK cell line according to an embodiment of the present invention, FIG. 10A is a graph showing the population doubling level when IL-2 was not added in the culture medium in the cultivation of NK101, NK101-7.28.CD, NK101-15.TN and NK111 cells, respectively; FIG. 10B is a series of histograms showing the results of flow cytometry analyses representing the expression of NKG2D in the NK101, NK101-7.28.CD, NK101-15.TN and NK111 cells, respectively; FIG. 10C is a series of graphs showing the results of flow cytometry analyses representing cell death ratios of various cancer cells (SK-MES-1, K562, A2780, Jeko-1, EoL-1, KG-1, IM9, Raji and HDLM-2) co-cultivated with NK101, NK101-7.28.CD, NK101-15.TN and NK111 cells, at E:T ratios of 1:1, 2:1, and 4:1, respectively, FIG. 10D is a series of graphs showing the results of cytotoxic analyses representing the change of cancer cell killing activity according to the treatment of TGF-β1 at various concentrations to co-cultivation of HDLM-2 (left) or IM9 (right) cells with NK101, NK101-7.28.CD, NK101-15.TN, and NK111 cells, respectively. As shown in FIG. 10A, through the introduction of membrane-bound IL-15, NK111 according to an embodiment of the present invention was able to proliferate independent of IL-2, which increases convenience during a production process. In addition, as shown in FIG. 10B, it was confirmed that the expression of NKG2D, a representative activating receptor of NK cells, was up-regulated by the introduction of IL-15 and thereby enhanced the cancer cell killing activity. Indeed, as shown in FIG. 10C, the killing ability against 9 cancer cell lines was enhanced through the introduction of IL-15. In addition, as confirmed in FIG. 10D, through the introduction of TGFβRIIΔcyto, a bait receptor for TGFβ1, an immunosuppressive factor that is over-secreted in the tumor microenvironment, the decrease of cancer killing ability by TGFβ1 was also not affected, which is result that is expected to be able to overcome the disadvantage of decreasing *in vivo* activity to some extent in the future.

Furthermore, the present inventors hypothesized that more effective anticancer treatment would be possible for cancers that highly express the corresponding cancer antigen if a chimeric antigen receptor capable of recognizing the cancer antigen specifically is transduced to the genetically modified NK cell line. Accordingly, a chimeric antigen receptor construct targeting FLT3, which is known to be overexpressed in various hematologic cancers, was designed (FIG. 7C), and then it was constructed and further transduced into the NK111 cell line prepared above. As a result, the FLT3-CAR-NK cells according to an embodiment of the present invention normally express the chimeric antigen receptor while maintaining the characteristics of the parental cell line (NK101) even during long-term passage (FIG. 11). In addition, as a result of performing a reactivity analysis using anti-FLT3-scFv-hyFc, it was confirmed that a specific binding reaction occurred against cancer cells overexpressing FLT3 (FIG. 12). Thus, the present inventors have completed the present invention by experimentally demonstrating that the FLT3-CAR-NK cell of the present invention exhibited very effective anticancer activity against FLT3 overexpressing cancer cells through *in vivo* experiments as well as *in vitro* experiments (FIGs. 13 to 16).

### MODE FOR INVENTION

Hereinafter, the present invention will be described in more detail through Examples and Experimental Examples. However, the present invention is not limited to the Examples and Experimental Examples described below, but may be implemented in various other forms. The following Examples and Experimental Examples are provided to enable the disclosure of the present invention to be complete and to fully convey the scope of the invention to those skilled in the art to which the present invention belongs.

### Example 1: Establishment of the NK cell line of the present invention

In order to establish a new NK cell-derived cell line, the following process was performed. Patient-derived extra-nodal NK lymphoma was placed on a 40 µm strainer, and 10 mL of Cellgro^{™} stem cell growth medium (SCGM, CellGenix, Germany, hereinafter referred to as 'NK media') supplemented with 20% fetal bovine serum (GE Healthcare, USA) and 1% antibiotics (Gibco, USA) was added, and then separated into single cells using the shear force of the piston of a 5 mL syringe and then suspended. NK cells from a single cell suspension were isolated using an NK isolation kit (Milltenyi Biotec, Germany), and the isolated NK cells were cultured in the NK media supplemented with 1,000 U/mL of recombinant human IL-2 (recombinant human IL-2 (rhIL-2), Prometheus Laboratories Inc., USA) for 3 weeks. NK media containing IL-2 was added twice a week, and the dividing cell line was continuously cultured until passage 30, confirming that a stable cell line was formed (FIG. 1A). Since the cell line expresses CD56 without expressing CD3, CD20, and CD16, it was confirmed that the origin of the cell was NK cells (FIG. 1B). It was confirmed under a microscope that the cell line had the characteristic of forming a spheroid when cultured (FIG. 1C), and NK101 cells were characteristic of large granular lymphocytes in the morphological analysis using the Wright-Giemsa staining method (FIG. 1D). In addition, it was confirmed that NK101 expresses cell death factor, perforin (green) and granzyme B (red) which are characteristics of NK cells through fluorescence staining) (FIG. IE). Co-culture was performed with K562, an MHCI negative cell sensitive to NK cells. After seeding K562 cells labeled with carboxyfluorescein diacetate (CFDA; Invitrogen, USA) in a 24-well plate (well-plate; Corning, USA) at a concentration of 3×10⁵ cells/mL, NK101 cells were treated with various effector cells versus targets. After being suspended in 1 mL medium at various E:T ratios of 1:1, 2:1, 4:1, and 10:1, respectively, the cancer cells were co-cultured with the NK cells for 24 hours (FIG. IF). After culturing, all cells were recovered from each well and centrifuged, and the cell pellet was suspended in FACS buffer. The cell pellet formed by centrifugation again was suspended in 100 µL of FACS buffer containing 1 µL LIVE/DEADFixable^{®} Near-IR Dead Stain Kit (Invitrogen, USA), and reacted at 4°C for 20 minutes. After washing twice with FACS buffer, the cell pellet was suspended in a solution obtained by diluting 5 µL of Annexin V APC (Biolegend, USA) in 100 µL of 1X Annexin V binding buffer, followed by reaction at room temperature for 20 minutes. Cell death was determined using flow cytometry for viable cells (Annexin V-negative/LIVE/DEAD-negative), early apoptotic cells (Annexin V-positive/LIVE/DEAD-negative), and late apoptotic cells (Annexin V-positive/ LIVE/DEAD-positive) and necrotic cells (Annexin V-negative/LIVE/DEAD-positive). From the results of FIGs. 1A to 1F, it was found that NK101 is an immortalized cell capable of continuous passage, forms a colony in culture, and has characteristics consistent with the phenotype and function of previously known NK cells. The cell line was designated as 'NK101' by confirming the cell lineage of the cell and fundamental characteristics of NK cells and deposited the novel NK cell line at the Korean Cell Type Culture under the Korea Research Institute of Bioscience and Biotechnology, located at 181 Ipsin-gil, Jeongeup-si, Jeollabuk-do, Republic of Korea on August 7, 2017 and was given an accession number of KCTC 13305BP on August 24, 2017. The depository is an international depository under the Budapest Treaty.

### Example 2: Analysis of cell proliferative ability of NK101

For establishment of culture conditions and comparison of division capacity for the NK101 cell line prepared in the Example 1, various concentrations of IL-2 in SCGM culture medium supplemented with 20% fetal bovine serum were treated to NK101 and NK-92 cells as a control. After treatment, the cell growth of the two cell lines was compared by MTS analysis. As shown in FIG. 2A, the growth of NK101 cell line was started at an IL-2 concentration of about 8 pM and growth was saturated at 500 pM (EC₅₀=23.3 pM), and the growth of NK-92 cell line was confirmed in the presence of IL-2 at a concentration of 30 pM and growth of NK-92 cells was stagnant at a concentration of 2000 pM (EC₅₀=128.3 pM), suggesting that NK101 requires a lower concentration of IL-2 than NK-92 for cell growth. Thus the present inventors investigated the expression of IL-2 receptor subunit which was confirmed in FIG. 2B by flow cytometry and it was confirmed that NK101 expressed high affinity IL-2 receptor CD25 compared to NK-92. In addition, as a result of analyzing the cell growth rate and viability of NK101 and NK-92 after freezing under the same culture conditions, NK101 recovered cell growth 2 days after thawing (passage 1), whereas NK-92 recovered cell growth 10 days (passage 5) after thawing. Thereafter, it was confirmed that a constant cell growth was reached (FIG. 2C) in NK101 cell line. After the cell growth of the two cells was stabilized, when the cell proliferation of the two cells was compared, it was confirmed that the total number of NK101 cells obtained after passage for 16 days was about 100 times the expected total number of NK-92 cells (FIG. 2D). This indicates that the productivity of the NK101 cells of the present invention is superior to that of the conventional NK-92 cells, suggesting that the NK101 cells of the present invention are very advantageous from an economic point of view.

The comprehensive characteristics of the NK101 cells of the present invention are summarized in Table 1 below.

**Table 1**

| Major characteristics of the NK101 cell line of the present invention | |
|---|---|
| **Features** | **NK101 cell line** |
| **Clinical data** | |
| Age/Sex | 56 yr./male |
| Race | Asian |
| Diagnosis | Extra-nodal NK/T lymphoma |

| **Cell culture** | |
|---|---|
| Growth pattern | Formation of multiple cell aggregates in suspension |
| Doubling time | 18-32 hrs |
| Maximum cell concentration | 1.2×10⁶ cells/mℓ |
| Minimum cell concentration | 0.5×10⁵ cells/mℓ |
| Cytokine dependency | IL-2 dependent(500 IU/mℓ) |
| Optimal division | every 2-3 days |

| **Immunological Features** | |
|---|---|
| T/NK markers | CD2⁺, CD3⁻, CD4⁻, CD7⁻, CD8⁻, CD16⁻, CD56⁺ |
| B cell markers | CD10⁻, CD19⁻, CD20⁻ |
| Myeloid monocyte markers | CD13⁻, CD14⁻, CD33⁺ |
| NK cell activating receptors | NKp46⁺, NKp30⁺, NKG2D⁺ |
| NK cell inhibiting receptors | KIR2DL1⁻, KIR2DL2⁻, ILT2⁻ |
| Descendent/activation markers | CD34⁻, FAS⁺ |
| Adhesion markers | ICD11a⁺, CD18⁺, CD54⁺ |

| **Functional features** | |
|---|---|
| NK activity | Secretion of pro-inflammatory cytokines and proliferative capacity |
| Cytokine production | Secretion of pro-inflammatory cytokines such as IFN-γ, TNF-α, GM-CSF, and IL-2 and non-secretion of IL-1 receptor antagonists and anti-inflammatory cytokines such as IL-10 |
| Cytokine receptors | CD25⁺, CD122⁺, CD132⁺, CD127⁻ |
| Chemokine receptors | CCR4⁺, CCR6⁺, CCR7⁺, CCR8⁺, CXCR3⁺, CXCR4⁺ |

### Example 3: Surface Marker Analysis

The NK101 cell line of the present invention prepared in Example 1 has the characteristics of floating cells, and the expression level of the surface antigens of the cells was confirmed by flow cytometry. The antibodies used were as follows:
Phycoerythrin (PE)-conjugated anti-CDla antibody (clone HI149), PE-conjugated anti-CD2 antibody (clone RPA-2.10), PE-conjugated anti-CD3 antibody (clone UCHT1), PE-conjugated anti-CD4 Antibody (clone SK3), PE-conjugated anti-CD5 antibody (clone UCHT2), PE-conjugated anti-CD7 antibody (clone CD7-6B7), PE-conjugated anti-CD8 antibody (clone RPA-T8), PE-conjugated anti - CD16 antibody (clone B73.1), PE-conjugated anti-CD25 antibody (clone BC96), PE-conjugated anti-CD28 antibody (clone CD28.2), PE-conjugated anti-CD56 antibody (clone HCD56), PE- Conjugated anti-CD57 antibody (clone HNK-1), PE-conjugated anti-CD94 antibody (clone DX22), PE-conjugated anti-CD122 antibody (clone TU27), PE-conjugated anti-CD132 antibody (clone TUgh4), PE- Conjugated anti-CD158a antibody (clone HP-DM1), PE-conjugated anti-CD158b antibody (clone DX27) and PE-conjugated anti-CD161 antibody (clone HP-3G10) were all purchased from Biolegend (San Diego, CA), and all of these antibodies were used in combination with LIVE/DEAD Fixable Near-IR dye (Molecular Probe, Eugene, OR).

For the expression analysis of NK-cell activating/inhibitory receptors or inhibitory immune checkpoints/PD-L1 on engineered NK101 cell lines, cells were stained with PE-conjugated anti-NKp44 (clone P44-8), -NKp46 (clone 9E2), -NKp80 (clone 5D12), -DNAM1 (clone 11A8), -NKG2D (clone 1D11), -NKp30 (clone P30-15), -2B4 (clone C1.7), -ILT2 (clone GHI/75), -KIR2DL1/S1/S3/S5 (clone HP-MA4), -KIR2DL2/DL3 (clone DX27), -PD-1 (clone NAT105), -CTLA4 (clone L3D10), -LAG-3 (clone 11C3C65), -TIM-3 (clone F38-2E2), - TIGIT (clone A15153G) or -PD-L1(clone 29E.2A3) antibody, all of which were purchased from Biolegend in combination with LIVE/DEAD Fixable Near-IR dye.

As a result of the flow cytometry analyses, in the case of T/NK cell markers, the NK101 cell line of the present invention expressed CD2 and CD56, which are surface antigens of NK cells, but does not express CD16, and the cell line did not express CD3, CD4, CD8, TCRαβ, and TCR which are surface antigens of T cells and CD20, a B cell surface antigen and CD14, monocyte surface antigen, thus the NK101 cell line was found to have characteristics of NK cells (FIG. 3A). In addition, the NK101 cell line of the present invention expressed NKG2D, NKp30, NKp46, DNAM-1, 2B4, but did not express NKp44 among NK cell activating receptors, whereas the NK101 cell line expressed CD94 and NKG2A, but did not express KIR2DL1/S1/S3/S5, KIR2DL2/DL3, and CD85j among NK cell inhibiting receptors (FIG. 3B). In addition, the NK101 cell line expressed CD2, CD11a, CD19, and ICAM-1, but did not express CD7, among cell adhesion molecules (FIG. 3C). Additionally, the NK101 cell line of the present invention expressed CD107a, perforin, granzyme B, and low levels of TRAIL, which are involved in cytotoxicity and immune activation of NK cells, but did not express FASL (FIG. 3D). Further, it was confirmed that IL-2 high affinity receptor CD25, and CD122 and CD132 among IL-2 receptors and IL-15Ra which is an IL-15 receptor, were expressed among cytokine receptors of NK cells (FIG. 3E). Among the chemokine receptors involved in NK cell line mobility, CCR4, CCR6, CCR7, CXCR3, and CXCR4 were expressed, but other CCRs and CXCRs were not expressed (FIG. 3F). In conclusion, the NK101 cell line of the present invention shows an activated NK cell phenotype, and is distinguished from other NK cell lines in that CD25 is particularly highly expressed. (Clausen, J. et al., Immunobiology, 207(2): 85-93, 2003), it can be seen that the NK101 cell line of the present invention is a very suitable cell line for mass production of cell therapeutics.

The expression of various cell markers in the NK101 cells of the present invention is summarized in Table 2 below.

**Table 2**

| Marker properties of NK101 cells of the present invention | | | |
|---|---|---|---|
| **Antigens** | **Exp.** | **Antigens** | **Exp.** |
| **Lineage markers** | | **Functional markers** | |
| CDla | - | CD95 (FAS) | +++ |
| CD2 | +++ | CD178 (FAS-L) | + |
| CD3 | - | CD107a | +++ |
| CD4 | - | TRAIL (CD253) | + |
| CD5 | - | Perforin | +++ |
| CD8 | - | Granzyme B | +++ |
| CD10 | - | IFNγ | +++ |
| CD11a | +++ | **Chemokine receptors** | |
| CD11c | - | CCR1 | + |
| CD13 | + | CCR2 | - |
| CD14 | + | CCR3 | - |
| CD16 | - | CCR4 | +++ |
| CD18 | +++ | CCR5 | + |
| CD19 | - | CCR6 | +++ |
| CD23 | - | CCR7 | +++ |
| CD33 | +++ | CCR8 | ++ |
| CD45 | +++ | CCR9 | + |
| CD56 | +++ | CXCR1 | - |
| CD57 | - | CXCR2 | - |
| CD161 | ++ | CXCR3 | +++ |
| **Activating receptors** | | CXCR4 | +++ |
| 2B4 | +++ | CXCR5 | - |
| NKp30 | ++ | CXCR6 | - |
| NKp46 | +++ | CXCR7 | - |
| NKG2D | ++ | **Cytokine Receptors** | |
| **Inhibiting receptors** | | CD25 (IL-2Ra) | +++ |
| CD85j (ILT2) | - | CD122 (IL-2Rb) | ++ |
| CD94 | +++ | CD132 (common γ chain) | +++ |
| CD158 (KIR2DL1/S1/S3/S5) | - | CD127 (IL-7Ra) | - |
| CD158b (KIR2DL2/DL3) | - | **Other markers** | |
| CD159a | +++ | TCRαβ | - |
| **Adhesion molecules** | | TCRγδ | - |
| DNAM-1 (CD226) | +++ | | |
| ICAM-1 (CD54) | +++ | | |
| CD62L | ++ | | |
| -, negative; +, < 10% positive; ++, 10-69% positive; +++, 70-100% positive (% means ratio of positive cells in the cell population) | | | |

### Example 4: Confirmation of CD56 and CD62L expression patterns of NK101

In order to analyze the characteristics of NK101 cells, the expression patterns of CD56 and CD62L, markers of NK cells, were analyzed and compared with those of NK-92 and primary NK cells using flow cytometry. As shown in FIG. 4A, NK101 cells were identified as CD56^{dim} NK cells with a lower level of CD56 expression compared to NK-92 cells. In addition, as shown in the contour graph of FIG. 4B, NK101 cells highly express the CD62L marker, which is not expressed in NK-92 cells, but is a marker that is limitedly expressed in some primary NK cells. In general, primary NK cells can be classified into CD56^{dim} and CD56^{bright} according to the expression pattern of CD56. CD56^{bright} NK cells have high cell proliferative capacity, secrete IFN-γ upon activation by cytokines, and show low cancer cell killing capacity, whereas CD56^{dim} NK cells have low proliferative capacity on the contrary to CD56bright NK cells, secretes IFN-γ upon recognition by target cells and shows high level of cytotoxicity. Recently validated CD56^{dim}CD62L⁺ NK cells (Juelke, K. et al., Blood, 116(8): 1299-1307, 2010; Luetke-Eversolh, M. et al., Front. Immunol., 4: 499, 2013) was reported as multi-functional NK cell line with both characteristics of CD56^{bright} and CD56^{dim} NK cells. It was confirmed that NK101 cells can proliferate and secrete IFN-γ by cytokine stimulation (FIG. 5A), and secrete various types of cytokines even upon recognizing target cells (FIG. 5B). In conclusion, the NK101 cell line has the phenotype and characteristics of CD56^{dim}CD62L⁺ NK cells, and although there have been reports of primary NK cells having CD56^{dim}CD62L⁺ phenotype or ex *vivo* expanded primary NK cells, the NK101 cell line is discriminated from the prior primary NK cells in that it is an immortalized NK cell line. In addition, it can be said that this is a unique characteristic that cannot be found among known NK cell lines.

### Example 5: Investigation of cancer cell killing activity and cytotoxicity mechanism in vitro of NK101

In order to confirm the cancer cell killing activity of the NK101 cell line prepared in the Example 1 and whose phenotype was identified, the following experiment was performed. Specifically, CFDA-labeled human-derived cancer cell lines THP-1, KG-1, HL-60 (acute myeloid leukemia), HCT116 (colon cancer), U373 (brain cancer), A2780 (ovarian cancer), A549 (lung adenocarcinoma), and SK-BR3 (breast cancer) cells were each seeded in a 24-well plate at a concentration of 3×10⁵ cells/mL by 1 mL. Thereafter, NK101 cells and control cells were suspended in 1 mL medium at various effector cell to target cell ratios (E:T ratio=1:1, 2:1, and 4:1), and then co-cultivated with the cancer cells for 24 hours. After co-culturing, all cells were collected, and recovered from each well, centrifuged, and the cell pellet was suspended in FACS buffer. The cell pellet formed by centrifugation was resuspended in 100 µL of FACS buffer diluted with 1 µL LIVE/DEAD^{®} Fixable Near-IR Dead Stain Kit, and reacted at 4°C for 20 minutes. After washing twice with FACS buffer, the cell pellet was resuspended in a solution obtained by diluting 5 µL of Annexin V APC (Biolegend, USA) in 100 µL of 1X Annexin V binding buffer, followed by reaction at room temperature for 20 minutes. Cell death ratio was determined using flow cytometry for viable cells (Annexin V-negative/LIVE/DEAD-negative), early apoptotic cells (Annexin V-positive/LIVE/DEAD-negative), and late apoptotic cells (Annexin V-positive/ LIVE/DEAD-positive) and necrotic cells (Annexin V-negative/LIVE/DEAD-positive). As shown in FIG. 6A, it was confirmed that the NK101 cell exhibited cell killing activity against various human cancer cell lines. After treatment with neutralizing antibodies against CD25, CD62L, DNAM-1, and CD54 (ICAM-1), which are highly expressed in NK cells, the NK01 cells were co-cultivated with THP-1 (FIG. 6B), K562 (FIG. 6C) and Jurkat (FIG. 6D) at an E:T ratio of 4:1, apoptosis was analyzed. As a result, it was confirmed that the apoptotic activity of NK101 against cancer cells was reduced by treatment with neutralizing antibodies against DNAM-1 and CD54, in case of THP-1 cells, with neutralizing antibodies against CD54, in case of K562, and with neutralizing antibodies against CD25, CD62L and CD54, respectively, in case of Jurkat cells. In addition, as shown in FIG. 6E, it was confirmed that, when the NK101 cells were co-cultured with THP-1, the apoptotic ability was synergistically reduced when the neutralizing antibodies against DNAM-1 and CD54 were co-treated. Based on the results, it is suggested that markers such as DNAM-1 and CD54 are mainly involved in the cancer cell killing activity by NK101 cells.

### Example 6: Preparation of Enhanced Functional NK Cell Line

Among the previously constructed NK cell lines, KHYG-1 and NK-92 are known to have a high ability to kill cancer cells, and they have the characteristic of expressing CD7 and/or CD28 in common, whereas NK101 does not express any of the two co-stimulatory factors (FIG. 8A). Accordingly, the present inventors tried to evaluate whether the cancer cell killing activity can be enhanced when the NK101 cells are transduced with the genes encoding CD7 and CD28.

### 6-1: Preparation of gene construct for transducing NK cell activating factors and cell suiced gene

The present inventors prepared a lentivirus transfer vector for the expression of transgenes, pBD-7.28.CD by constructing a gene construct in which a polynucleotide (SEQ ID NO: 15) encoding CD7 (SEQ ID NO: 14) is linked to a polynucleotide (SEQ ID NO: 17) encoding CD28 (SEQ ID NOL: 16), an immune cell co-stimulatory factor (SEQ ID NO: 16) and further linked to a polynucleotide (SEQ ID NO: 21) encoding a fusion protein CD::UPRT via an IRES (SEQ ID NO: 22) and cloning the gene construct into a commercial lentivirus transfer vector pBD (SL-Bigen, Seongnam, Korea), in order to prepare a new NK cell line with enhanced function (FIG. 7A).

### 6-2: Preparation of lentiviruses

For preparing lentiviruses, 8.5×10⁶ Lenti-X 293T cells were plated into a 150-mm dish. Two days later, cells were transfected with 12 µg of pBD-7.28.CD transfer plasmid prepared in the Example 6-1, 12 µg of psPAX2 (Addgene plasmid, #12260) and 2.4 µg of pMD2.G (Addgene plasmid, #12259) using lipofectamine reagent (Thermo Fisher Scientific). Two days after transfection, virus containing supernatant was collected, filtered through a 0.45 µm pore size filter (Millipore, USA) and concentrated using Lenti-X Concentrator (Takara Bio). The virus pellet was resuspended in serum-free DMEM and stored at -80°C until further use.

As a final step, the lentivirus-containing concentrate was centrifuged at 4000 rpm for 60 minutes, the supernatant was removed, and the lentivirus recovered in the form of a pellet was diluted with 1-2 mL of culture solution and stored at -80°C until use.

### 6-3: Preparation of a NK cell line transduced with genes encoding NK cell activation factors and a cell suicide gene

Firstly, 2×10⁶ cells of NK101 cells resuspended in 1.5 mL were infected with the lentiviruses expressing pBD-7.28.CD prepared in the Example 6-2 by mixing the NK101 cells with lentiviruses (MOI = 10), in the presence of 8 mg/ml protamine sulfate (Sigma, USA), 6 µM BX795 (Invitrogen, USA) and 250 IU/ml of IL-2. The cell culture plate was centrifuged at 1,800 rpm at room temperature and then incubated at 37°C for 4 hours. Then, the same infection process was repeated once again and after overnight culture, the cells were centrifuged, resuspended in fresh culture medium, and cultured for 3 days before evaluation of transgene expression. After 72 hours of infection, the expression of CD7 and CD28, which are immune cell co-stimulatory factors, was confirmed by flow cytometry, and a week later, the FACSAria cell sorter and Cell Quest software (BD Biosciences, USA) were used to detect and isolate the infected cells. In addition, clones showing stable growth and high transgene expression as passages were repeated were selected for future analysis. The NK101 cells transduced with lentivirus expressing pBD-7.28.CD were stained with PE-conjugated anti-CD7 (Biolegend) and allophycocyanin (APC)-conjugated anti-CD28 (Biolegend) antibodies and sorted for the double-positive population (FIG. 8B). The present inventors selected the most suitable clone among isolated clone and designated the clone as "NK101-7.28.CD".

### 6-4: Reverse transcriptase polymerase chain reaction (RT-PCR)

The expression of CD::UPRT, which is an intracellular protein was confirmed using reverse transcription polymerase chain reaction (RT-PCR) using isolated total RNA (FIG. 8C). Total RNA was isolated using an RNA extraction kit (iNtRON, South Korea). For the reverse transcription polymerase chain reaction, cDNA was synthesized using QuantiTect Reverse Transcription Kit (QIAGEN, Germany), and the synthesized cDNA was mixed with *Taq* DNA polymerase and primers, and then polymerase chain reaction (PCR) was performed. The amplified PCR product was electrophoresed on a 1% agarose gel to confirm the presence of the CD::UPRT gene. As a result, as shown in FIG. 8C, it was confirmed that the transduced CD::UPRT gene was normally expressed.

### Example 7: Analysis of NK activation by transduced cell co-stimulators and a cell suicide gene

### 7-1: Verification of NK cell killing activity by introduction of CD7 and CD28

The cytotoxic activity of NK cells is regulated by several cell surface receptors that transmit activating or inhibitory signals (Wang and Matosevic, Cell Oncol. 43: 577-600, 2020). Since no inhibitory receptors are expressed on NK101 (Yang et al., J. Immunother. Cancer 7(1): 138, 2019), the present inventors investigated whether the cytolytic potential of NK101 by restoration of missing activating receptor expression can be improved. Accordingly, the present inventors first investigated the expression level of surface markers functionally used to define the NK cells in NK101 compared to KHYG-1 and NK-92, which possess potent cancer-killing activity (Klingemann et al., Front. Immunol. 7: 91, 2016). Through comparative flow cytometry analyses for 18 different surface markers, the present inventors found that two of the markers, CD7 and CD28, were expressed in one or both of the control cell lines but not completely in NK101 (FIG. 8A). Since it was suggested that CD7 and CD28 provide stimulatory signals in primary NK cells and NK cell lines, respectively, the present inventors evaluated whether forced expression of CD7 and CD28 in NK101 could enhance their cytotoxic function. To determine the effect of CD7 and CD28 expression on cytotoxicity of NK101 cells, unmodified NK101 and NK101-7.28.CD were co-cultivated with human tumor cell lines (FIG. 8D) which exhibit a broad spectrum of surface expression pattern of B7 molecule (CD80/CD86), a ligand of CD28 and SECTM1, ligand of CD7), and tumor cell apoptosis was measured by flow cytometry (FIG. 8F). Compared to the parental cell line NK101, NK101-7.28.CD was showed almost the same cytotoxicity against B7⁻ tumor cells, including SK-MES-1, K562 and A2780. On the other hand, NK101-7.28.CD showed significantly stronger cell killing activity against B7⁺ tumor cells. The degree of increase in cytotoxicity following CD7/CD28 insertion was related to (i) the co-existence of CD80 and CD86 on the surface of tumor cells as shown in the fact there was a larger difference in the specific lysis rates against CD80⁺CD86⁺ cancer cells between NK101 and NK101-7.28.CD cell lines, and (ii) the degree of expression of CD86 on the surface of cancer cells, as shown in the fact that NK101-7.28.CD cells exhibited a greater cytotoxic effect in HDLM-2 cells (MFI=17,463) than in Raji cells (MFI=9,265) or IM-9 cells (MI=8, 937) . The present inventors could not observe a clear correlation between the degree of lysis of the target cells and the surface SECTM1 expression of the target cancer (FIGs. 8D and 8E). Collectively, these results suggest that the enhanced oncolytic activity of NK101-7.28.CD is mainly mediated by intercellular interactions between CD28 and ligands expressed on target cells.

### Example 8: Introduction of mbIL-15 and TGFPRIIΔcyto

### 8-1: Preparation of mbIL-15 and TGFPRIIΔcyto co-expression gene construct

The present inventors prepared a lentivirus transfer vector pBD.15.TN by constructing a gene construct in which a polynucleotide (SEQ ID NO: 24) encoding a membrane-bound IL-15 (mbIL-15, SEQ ID NO: 23) is linked to a polynucleotide (SEQ ID NO: 26) encoding TGFβRIIΔcyto (SEQ ID NO: 25) from which the cytoplasmic domain of TGFβRII has been removed via a polynucleotide (SEQ ID NO: 19) encoding a 2A peptide (SEQ ID NO: 19) and cloning the gene construct into a commercial lentivirus transfer vector, pBD, in order to improve cell killing activity of the NK101-7.28.CD cells prepared in the Example 6 (FIG. 7B).

### 8-2: Lentivirus Preparation

For preparing lentiviruses, 8.5×10⁶ Lenti-X 293T cells were plated into a 150-mm dish. Two days later, cells were transfected with 12 µg of pBD-15.TN transfer plasmid prepared in the Example 8-1, 12 µg of psPAX2 (Addgene plasmid, #12260) and 2.4 µg of pMD2.G (Addgene plasmid, #12259) using lipofectamine reagent (Thermo Fisher Scientific). Two days after transfection, virus containing supernatant was collected, filtered through a 0.45 µm pore size filter (Millipore, USA) and concentrated using Lenti-X Concentrator (Takara Bio). The virus pellet was resuspended in serum-free DMEM and stored at -80°C until further use.

As a final step, the lentivirus-containing concentrate was centrifuged at 4000 rpm for 60 minutes, the supernatant was removed, and the lentivirus recovered in the form of a pellet was diluted with 1-2 mL of culture solution and stored at -80°C until use.

### 8-3: Preparation of a NK cell line in which genes encoding mbIL-15 and TGFβRIIΔcyto were transduced

IL-15 is a pleiotropic cytokine that increases NK cell proliferation and effector function (Carson et al., Braz. J. Med. Biol. Res. 31: 1-9, 1998). Membrane-bound IL-15 (mbIL-15), a major physiological form of cytokine, is known to transmit a stronger activation signal than soluble IL-15 (van Belle et al., Arch. Immunol. Ther. Exp. (Warsz) 53: 115-126, 2005; Cho et al., Kor. J. Lab. Med. 29: 89-96, 2009). To evaluate the effect of mbIL-15 expression on the cytotoxicity of NK101, the present inventors infected NK101 and NK101-7.28.CD cells with the lentivirus prepared using pBD.15.TN prepared in Example 8-2.

Firstly, 2×10⁶ cells of each NK101 and NK101-7.28.CD cell line resuspended in 1.5 mL was infected with the lentiviruses expressing pBD-15.TN prepared in the Example 8-2 by mixing the cells with lentiviruses (MOI = 10), in the presence of 8 mg/ml protamine sulfate (Sigma, USA), 6 µM BX795 (Invitrogen, USA) and 250 IU/ml of IL-2. The cell culture plate was centrifuged at 1,800 rpm at room temperature and then incubated at 37°C for 4 hours. Then, the same infection process was repeated once again and after overnight culture, the cells were centrifuged, resuspended in fresh culture medium, and cultured for 3 days before evaluation of transgene expression. After 72 hours of infection, the expression of mbIL-15 and TGFβRIIΔcyto was confirmed by flow cytometry, and a week later, the FACSAria cell sorter and Cell Quest software (BD Biosciences, USA) were used to detect and isolate the infected cells. The selected clones were designated as "NK101-15.TN" and "NK111", respectively (FIGs. 9A and 9B).

### 8-4: Analysis of tumor cell killing activity according to the introduction of mbIL-15

In order to investigate the effect according to the introduction of mbIL-15, the present inventors analyzed proliferation patterns of NK101, and NK101-7.28.CD as well as NK101.15.TN and NK111 cells in which mbIL-15 gene was transduced to the parent NK cells according to depletion of IL-2. As a result, similar to the results of previous studies on mbIL-15-introduced primary NK cells (Imamura et al., Blood 124: 1081-1088, 2014), NK101-15.TN or NK111 cells were stable without exogenous IL-2 whereas the parent NK101 cells or the NK101-7.28.CD cells failed to proliferate in the absence of IL-2 (FIG. 10A). For the next experiment, NK101-15.TN or NK111 cells were maintained in IL-2 deficient conditions. Then, parallel co-cultivations of NK101, NK101-7.28.CD, NK101-15.TN or NK111 cells with the tumor cells listed in Example 7-1 were performed, and the degree of apoptosis of cancer cells was measured through flow cytometry. In all co-cultivations, more improved tumor killing effect in the NK101-15.TN and NK111 cells according to the introduction of mbIL-15 was confirmed than each control group (NK101 and NK101-7.28.CD), as shown by higher ratio of specific cytolysis. The present inventors also confirmed that the degree of increase in cytotoxicity from NK101 to NK101-15.TN was similar to that from NK101-7.28.CD to NK111 in most co-cultures (FIG. 10C). As previous studies suggested that IL-15-mediated cytotoxicity improvement involves upregulation of NKG2D in stimulated NK cells, the present inventors compared surface NKG2D expression in NK101, NK101-7.28.CD, NK101-15.TN and NK111 cells by flow cytometry (FIG. 10B). As a result, the ratio of NKG2D⁺ cells in NK101 and NK101-7.28.CD was 36% and 51%, respectively. In particular, mbIL-15 introduction increased NKG2D expression, as shown in 69% and 98% of the NKG2D⁺ population in NK101-15.TN and NK111 cells, respectively (FIG. 10B). It was also noted that the expression levels of other activating receptors (DNAM-1, NKp46, NKp44, and NKp80) and inhibitory receptors (ILT2, KIR2DL1/S1/S3/S5, KIR2DL2/L3) remained as the same level in all four cell lines analyzed (FIG. 10E). Overall, the introduction of mbIL-15 not only conferred the ability to grow and survive autonomously, but also increased the cytolytic activity of NK101 through upregulation of surface NKG2D expression.

### 8-5: Inhibition of immunosuppression by inherent TGFβ by the introduction of TGFβRIIΔcyto

In addition, the present inventors have investigated whether the immunosuppressive effect of TGFβI, which is well known as an immunosuppressive factor present in large amounts in a patient's body, is inhibited by TGFβRIIΔcyto introduced into NK111 cells. For this purpose, HDLM-2 and Raji cells which are target tumor cells and 3×10⁵ cells/ml of NK101, NK101-7.28.CD, NK101-15.TN and NK111, which are effector cells in 24-well plates with or without TGFβ (3, 10 and 30 ng/mL) were seeded in an ET ratio of 1:1, and co-cultivated for 24 hours at 5% CO², 37°C, the ability to kill cancer cells was investigated through flow cytometry (FIG. 10D). As a result, as shown in FIG. 10D, the cytotoxic activity of NK101 and NK101-7.28.CD was reduced and the functions of NK101-15.TN or NK111 were not affected by TGFβ treatment at all doses tested. These results suggest that TGFβ signaling is effectively blocked by the introduction of TGFβRIIΔcyto and that transduced NK101 cells can reverse the immunosuppressive effect of TGFβ.

As described above, the NK101-7.28.CD cells according to an embodiment of the present invention significantly increased cancer cell killing activity compared to the parental cell line NK101 while maintaining its main characteristics, and the NK101-7.28.CD NK111 cells with enhanced performance not only significantly increased cancer cell killing activity compared to NK101-7.28.CD cells, but also could avoid inhibition of cell killing activity due to TGFβ, so it is expected that it can be used as a very effective cancer treatment when administered *in vivo.*

### Example 9: Preparation of an NK cell line in which a chimeric antigen receptor targeting FLT3 is introduced

The present inventors investigated whether antigen-specific cancer cell killing activity is enhanced when a polynucleotide encoding a chimeric antigen receptor (hereinafter, abbreviated as 'CAR') is transduced into the NK111 cell line prepared in Example 8. Specifically, the present inventors prepared a FLT3 targeting chimeric antigen receptor gene construct and then transduced it into NK111 to prepare NK111-CAR cells and evaluated their functions.

### 9-1: Construction of anti-FLT3 scFv-CAR gene construct

In order to construct a tumor-targeting NK cell line, the present inventors developed prepared a lentivirus transfer plasmid, pBD-CAR for expressing a CAR targeting FLT3 by preparing a gene construct comprising a polynucleotide (SEQ ID NO: 12) encoding an anti-FLT3 scFv-CAR protein (SEQ ID NO: 11) which comprises an scFv targeting FLT3 (SEQ ID NO: 13), a modified human immunoglobulin (Ig) Fc region (SEQ ID NO: 1), CD28 transmembrane domain (SEQ ID NO: 3), DAP10 intracellular activation domain (SEQ ID NO: 5), DAP12 intracellular activation domain (SEQ ID NO: 7), and CD3ζ cytoplasmic signaling domain (SEQ ID NO: 9) and cloning the gene construct into a commercial transfer vector pBD for lentivirus production (FIG. 7C).

### 9-2: Preparation of lentiviruses expressing anti-FLT3 scFv-CAR

For preparing lentiviruses, 8.5×10⁶ Lenti-X 293T cells were plated into a 150-mm dish. Two days later, cells were transfected with 12 µg of pBD-CAR transfer plasmid prepared in the Example 9-1, 12 µg of psPAX2 (Addgene plasmid, #12260) and 2.4 µg of pMD2.G (Addgene plasmid, #12259) using lipofectamine reagent (Thermo Fisher Scientific). Two days after transfection, virus containing supernatant was collected, filtered through a 0.45 µm pore size filter (Millipore, USA) and concentrated using Lenti-X Concentrator (Takara Bio). The virus pellet was resuspended in serum-free DMEM and stored at -80°C until further use.

As a final step, the lentivirus-containing concentrate was centrifuged at 4000 rpm for 60 minutes, the supernatant was removed, and the lentivirus recovered in the form of a pellet was diluted with 1-2 mL of culture solution and stored at -80°C until use.

### 9-3: Preparation of a NK cell line in which a gene encoding anti-FLT3 scFv-CAR was transduced

Chimeric antigen receptor modification is an effective method of conferring tumor antigen specificity to NK cells lacking intrinsically rearranged antigen receptors (Li et al., Cancer Gene Ther. 17: 147-154, 2010; Wu and Wang, Cell. Mol. Immunol. 15: 1095-1097, 2018). Thus, NK111 cells were infected with the lentivirus prepared using the pBD-CAR prepared in Example 9-2.

Firstly, 2×10⁶ cells of NK111 cell line resuspended in 1.5 mL was infected with the lentiviruses expressing pBD-CAR prepared in the Example 9-2 by mixing the cells with lentiviruses (MOI = 10), in the presence of 8 mg/ml protamine sulfate (Sigma, USA), 6 µM BX795 (Invitrogen, USA) and 250 IU/ml of IL-2. The cell culture plate was centrifuged at 1,800 rpm at room temperature and then incubated at 37°C for 4 hours. Then, the same infection process was repeated once again and after overnight culture, the cells were centrifuged, resuspended in fresh culture medium, and cultured for 3 days before evaluation of transgene expression. After 72 hours of infection, the expression of anti-FLT3 scFv-CAR was confirmed by flow cytometry, and a week later, the FACSAria cell sorter and Cell Quest software (BD Biosciences, USA) were used to detect and isolate the infected cells. The selected clones were designated as "NK111-CAR" (Left panel of FIG. 11A).

As a result of flow cytometry, as shown in FIG. 11A, it was confirmed that NK111 cells expressing anti-FLT3 scFv-CAR according to the present invention reacted specifically to anti-human Fc protein antibodies recognize the modified Fc region (SEQ ID NO: 1) encoded by the nucleotide sequence shown in SEQ ID NO: 2, indicating that the introduced CAR is successfully expressed on the cell surface.

In addition, in order to analyze the human FLT3 protein binding ability of anti-FLT3 scFv-CAR expressed on the cell surface, the following experiment was performed through FACS analysis. After washing the NK111 cell line expressing anti-FLT3 scFv-CAR (using 2.5×10⁵ cells) twice with FACS buffer, 4 µl of Human TruStain FcX (Fc Receptor Blocking Solution) (cat# 422302, Biolegend, USA) diluted in 100 µl of FACS buffer was treated with the transduced NK111 cells and incubated for 10 min at room temperature. After the reaction was completed, 15 µl of recombinant human FLT3-Fc chimeric protein (cat# 368-ST/CF, R&D, USA) was added to the FACS tube and reacted at 4°C for 20 minutes. After washing the unbound antibody with FACS buffer, 3 µl of anti-human IgG-PE (cat#: 409304, Biolegend, USA) secondary antibody was diluted in 100 µl of FACS buffer was added and incubated at 4°C for 20 minutes. After the reaction, the unbound antibodies were washed twice with FACS buffer, and then FACS analysis was performed (Right panel of FIG. 11A).

As a result, as shown in FIG. 11A, it was confirmed that NK111 cells expressing anti-FLT3 scFv-CAR according to the present invention bind to human FLT3-Fc protein. In addition, Western blot analysis of NK101, NK111, and NK111-CAR lysates using anti-CD3ζ antibody confirmed that CAR molecules were mainly expressed exclusively in the NK111-CAR cells in a dimeric configuration (FIG. 11B) .

### 9-4: Analysis of changes in antigen-specific cancer cell killing activity

In order to determine whether the CAR expressed on NK111 cells recognizes FLT3 on cancer cells, the present inventors analyzed whether a recombinant anti-FTL3-modified Fc fusion which is same as the anti-FLT3 scFv-CAR protein or the present invention except transmembrane domain and intracellular signal domains can bind to various cancer cells trough flow cytometry using an anti-FLT3 antibody. The preparation of the recombinant fusion protein was performed as follows:
HEK293-F cells were seeded in a 1 L shake flask (Corning) at a density of 1.0×10⁶ cells/mL in 200 mL, and cultured at 120 rpm, 8% CO₂ conditions. One day later, the cells transfected with about 21 ml of complex containing a total of 400 µg (2 µg/mL) of plasmid DNA encoding the anti-FLT3-scFv-Fc protein in 10 mL of FreeSytle 293 expression medium (Invitrogen) supplemented with 800 µg PEI (4 µg/mL), and the supernatant was harvested 5 days after transfection. The anti-FLT3 scFv-Fc construct was constructed by cloning only a polynucleotide encoding anti-FLT3 scFv (SEQ ID NO: 13) and a polynucleotide encoding modified Fc region (SEQ ID NO: 1) from the gene construct prepared in the Example 9-1.

Proteins were purified from cell culture supernatants harvested with reference to standard protocols. The antibody was applied to a Protein A Sepharose column (GE healthcare) and washed with PBS (pH 7.4). After eluting the antibody at pH 3.0 with 0.1 M glycine buffer, the sample was immediately neutralized with 1 M Tris buffer. The eluted antibody fraction was exchanged with PBS (pH 7.4) using a Pierce Dextran Desalting Column (5K MWCO), and then concentrated using a MILLIPORE Amicon Ultra (10 MWCO) centrifugal concentrator. The purified protein was quantified through BCA method.

The acute myeloid leukemia cell lines EOL-1, MOLM-13, and REH cell lines known to express FLT3 protein on the cell surface, as well as the FLT3-negative cell lines K562, Daudi and normal human bone marrow cells (2.5×0⁵ cells) were washed twice with FACS buffer. Then, 4 µl of Human TruStain FcX (Fc Receptor Blocking Solution) (cat# 422302, Biolegend, USA) diluted in 100 µl of FACS buffer was treated to the cells and reacted at room temperature for 10 minutes. After the reaction was completed, 1 µg of the prepared anti-FLT3-scFv-Fc protein was added to the FACS tube and reacted at 4°C for 20 minutes. After washing the unbound antibodies with FACS buffer, 3 µl of anti-human IgG-PE (cat#: 409304, Biolegend, USA) secondary antibody was diluted in 100 µl of FACS buffer was treated and incubated at 4°C for 20 minutes. After the reaction, the unbound antibodies was washed twice with FACS buffer, and then FACS analysis was performed. In the case of a commercialized anti-human FLT3 (CD135)-PE antibody (cat #313308, Biolegend, USA), after treatment with Fc receptor blocking solution, 3 µl was treated, reacted at 4°C for 20 minutes, and finally washed, followed by FACS analysis was performed.

As a result of the flow cytometry analysis, the anti-FLT3 scFv-hyFc fusion protein showed strong binding to FLT3⁺ EOL-1, MOLM-13, and REH cell lines like commercial antibodies, but it did not bind to FLT3⁻ K562, Daudi, and normal human bone marrow cells (FIG. 12). Subsequently, the present inventors investigated antigen-specific tumor cell killing activity by NK cell lines during co-culture of these cancer cells with NK111 or NK111-CAR cells. As expected, NK111-CAR cells showed stronger cytotoxicity against FLT3⁺ EOL-1, MOLM-13, and REH cells than the parental cell line NK111, whereas similar cytotoxicity was found against FLT3⁻ K562, Daudi and normal human bone marrow cells (FIG. 13). Collectively, these results are direct evidences for the specific recognition of CAR-modified NK11 cells to FLT3⁺ tumor cells and enhanced cytolytic activity for the tumor cells by CAR-modified NK111.

### Example 10: In vitro anticancer activity of NK111-CAR cells

In order to analyze the cell killing activity of the NK111-CAR cells prepared in Example 9-3, EOL-1 (acute myeloid leukemia), MOLM-13 (acute myeloid leukemia), REH (acute lymphocytic leukemia), and K562 (Chronic myeloid leukemia) and normal human bone marrow cells (normal BM) were co-cultured with the NK111-CAR cells. A target tumor cell line labeled with Celltracker violet dye (CTV; Invitrogen, USA) was prepared at a concentration of 3×10⁵ cells/mL, and then aliquoted by 1 mL into a 24-well plate. Thereafter, NK111 and NK111-CAR cells were resuspended at various effector cell to target cell ratios (E:T ratio=1:1, 2:1, 4:1), and then co-cultivated with the target tumor cells. After the co-cultivation, all cells were recovered from each well and centrifuged to obtain cell pellets. The cell pellets were resuspended in 100 µl of FACS buffer diluted with 1 µl LIVE/DEAD Fixable Near-IR Dead Stain Kit (Life Technologies), and reacted at 4°C for 20 minutes. After washing twice with FACS buffer, 5 µl of Annexin V APC (BioLegend, USA) was diluted in 100 µl of IX Annexin V binding buffer and stained at room temperature for 20 minutes. Cell death was classified into viable cells, early apoptotic cells, late apoptotic cells, and necrotic cells using flow cytometry (FIG. 13). As a result, as shown in FIG. 13, NK111-CAR cells showed significantly higher apoptotic activity in FLT3-expressing EoL-1, MOLM-13, and REH cells compared to the control group NK111, whereas there is no effect on FLT3-negative normal bone marrow cells similar as NK111. This means that NK111-CAR cells specifically recognize FLT3-expressing cancer cells.

### Example 11: Analysis of changes in NK111-CAR cell characteristics according to irradiation

Since the NK111-CAR cell line of the present invention is a cell in which the lentivirus is used for transduction of NK101, an NK lymphoma-derived cell line, in order to use it as a therapeutic agent, is necessary to suppresses cell growth through irradiation to eliminate tumorigenicity and it should maintain a degree of anticancer activity at the same time. To verify this, the present inventors evaluated whether cell growth inhibition and cancer cell killing activity after irradiating 0 Gy or 10 Gy of gamma radiation to the NK111-CAR cell line (FIGs. 15A and 15B).

As a result, as shown in FIG. 15A, it was confirmed that non-irradiated NK111-CAR cells showed a continuous proliferation pattern until day 4, whereas NK111-CAR cells irradiated with 10 Gy inhibited cell growth and all died after 72 hours. In addition, as shown in FIG. 15B, it was confirmed that the NK111-CAR cell line irradiated with 10 Gy of radiation showed significantly higher cell killing activity than control NK111 in FLT3 overexpressing cells EOL-1 and Molm-13, and the same killing ability was maintained even after irradiation. On the other hand, it was confirmed that the K562 cell line, which is FLT3-netative, showed a similar level of killing ability to the control NK111 both before and after irradiation. Therefore, it is suggested that the NK111-CAR cell line can be used for the purpose of removing cancer cells since it exhibits equivalent activity without *in vivo* proliferation even after irradiation.

### Example 12: In vivo anti-leukemia activity analysis of NK111-CAR cells and enhancement of anticancer efficacy by co-administration

The present inventors investigated the anticancer activity of the NK111-CAR cell line using AML xenograft tumor model mice in order to analyze the *in vivo* anticancer activity of the NK111-CAR. 4 days after intravenous inoculation of EoL-1-luc-EGFP 1×10⁶ cells, a human acute myeloid leukemia cell line expressing FLT3 and luciferase to 6-8 weeks old mice (NOD/SCID IL2Rγ^{null}, NSG), control (growth medium) and NK111-CAR cells irradiated with 10 Gy of 5×10⁶ cells were intravenously administered 5 times a week, for a total of 10 times at 24 hour intervals. And then, after subcutaneous administration of 150 mg/kg luciferin for the degree of bioluminescence by luciferase emitted from cancer cells, tumor growth was periodically monitored by bioluminescence imaging (BLI) using Lago (Spectral Imaging Instruments, Tucson, AZ) (FIG. 16A). In addition, two weeks after NK111-CAR administration, the experimental animals were sacrificed, and bone marrow, spleen, lung and liver were excised and treated as previously reported (Kim et al., Clin. Cancer Res. 19: 415-427, 2013). Flow cytometry was performed by staining single cell suspensions from various organs with an APC-conjugated anti-CD56 antibody (clone HCD56, Biolegend) and LIVE/DEAD Fixable Near-IR dye. Viable cells were gated and the proportion of residual tumor cells (GFP-positive) and NK111-CAR cells (PE-positive) was determined using the BD LSRFortessa system. For the repeat-dose study, EOL-1 tumor-bearing mice were injected with irradiated 5×10⁶ NK111-CAR cells by tail vein 3 times at weekly intervals for 2 weeks. All animal experiments were performed in accordance with the guidelines of the Laboratory Animal Care and Use Committee.

As a result, as shown in FIGs. 16B and 16C, it was confirmed that the NK111-CAR cells irradiated with 10 Gy showed a significant level of anti-leukemia inhibitory effect compared to the control group. In addition, in order to investigate whether the anticancer activity is enhanced according to the combination administration with the drug being used in clinical practice, the anticancer effect of NK111-CAR was analyzed by co-administration with LY2510924, a CXCR4 peptide antagonist. In the case of the combination with LY2510924, from the time of administration of the NK111-CAR cells, 2.5 mg/kg of LY2510924 was administrated intraperitoneally 5 times a week, at an interval of 24 hours, in a total of 10 administrations. As a result, it was confirmed that the efficacy of NK111-CAR was more enhanced when administered in combination with other anticancer agent than when NK111-CAR was administered alone.

Formulation examples for the composition of the present invention are exemplified below.

### Formulation Example 1: Preparation of injections

Recombinant NK111-CAR cells 1×10⁸ to 5×10¹² cells
Appropriate amount of pH adjuster
Appropriate amount of stabilizer
Up to 100% sterile distilled water for injection

According to a conventional method for preparing injections, the content of the above ingredients per 1 ampoule (2 ml) was prepared.

Although the present invention has been described with reference to the above-described examples and experimental examples, it will be understood that these are merely exemplary, and that various modifications and equivalent other embodiments are possible therefrom by those skilled in the art. Therefore, the true technical protection scope of the present invention should be determined by the technical spirit of the appended claims.

### Industrial Applicability

The genetically modified immune cell line according to an embodiment of the present invention can be usefully used in the manufacture of anticancer cell therapeutic products.

## Claims

1. A genetically modified NK cell line expressing a FLT3-specific chimeric antigen receptor by transducing an isolated NK cell line having following characteristic with a polynucleotide encoding an FLT3-specific chimeric antigen receptor:
positive for CD2, CD7, CD11a, CD25, CD28, CD45, CD54, DNAM-1, CD62L and CD56; and
negative for CD1a, CD3, CD4, CD8, CD14, CD20, CD23, CD34, TCRαβ and TCRγδ.

2. The genetically modified NK cell line according to claim 1, further transduced with a cell suicide gene.

3. The genetically modified NK cell line according to claim 2, wherein the cell suicide gene is uracil phosphoribosyl transferase (UPRT) gene, herpes simplex virus thymidine kinase (HSV TK) gene, varicella zoster virus thymidine kinase (VZV TK) gene, cytosine deaminase (CD) gene, a gene encoding a fusion protein (CD::UPRT) of the CD and UPRT, a carboxyl esterase gene, a nitroreductase gene, a carboxypeptidase G2 gene, or an inducible caspase 9 (iCas9) gene.

4. The genetically modified NK cell line according to claim 3, wherein The CD::UPRT comprises the amino acid sequence represented by SEQ ID NO: 20.

5. The genetically modified NK cell line according to claim 1, further transduced with a polynucleotide encoding membrane-bound IL-15 (mbIL-15).

6. The genetically modified NK cell line according to claim 5, wherein the mbIL-15 comprises the amino acid sequence represented by SEQ ID NO: 23.

7. The genetically modified NK cell line according to claim 1, further transduced with a polynucleotide encoding a cytoplasmic domain deletion TGFβ receptor II (TGFβRIIΔcyto).

8. The genetically modified NK cell line according to claim 7, wherein the TGFβRIIΔcyto comprises the amino acid sequence represented by SEQ ID NO: 25.

9. The genetically modified NK cell line according to claim 1, further transduced with a cell suicide gene, a polynucleotide encoding a membrane-bound IL-15, and a polynucleotide encoding a TGFβRII variant whose cytoplasmic domain is deleted.

10. The genetically modified NK cell line according to claim 1, wherein the FLT3-specific chimeric antigen receptor comprises an scFv that specifically binds FLT3, a modified Ig Fc domain, a CD28 transmembrane domain, a DAP10 intracellular activation domain, a DAP12 intracellular activation domain and a CD3ζ cytoplasmic signaling domain of a T cell receptor.

11. The genetically modified NK cell line according to claim 10, wherein the scFv comprises the amino acid sequence represented by SEQ ID NO: 13.

12. The genetically modified NK cell line according to claim 10, wherein the modified Ig Fc domain comprises the amino acid sequence represented by SEQ ID NO: 1.

13. The genetically modified NK cell line according to claim 10, wherein the CD28 transmembrane domain comprises the amino acid sequence represented by SEQ ID NO: 3.

14. The genetically modified NK cell line according to claim 10, wherein the DAP10 intracellular activation domain comprises the amino acid sequence represented by SEQ ID NO: 5.

15. The genetically modified NK cell line according to claim 10, wherein the DAP12 intracellular activation domain comprises the amino acid sequence represented by SEQ ID NO: 7.

16. The genetically modified NK cell line according to claim 10, wherein the CD3ζ cytoplasmic signaling domain of the T cell receptor comprises the amino acid sequence represented by SEQ ID NO: 9.

17. The genetically modified NK cell line according to claim 1, wherein the FLT3-specific chimeric antigen receptor comprises the amino acid sequence represented by SEQ ID NO: 11.

18. A cell therapeutic agent for treating cancer containing a therapeutically effective amount of the genetically modified NK cell line of any one among claims 1 to 17 as an active ingredient.

19. The cell therapeutic agent according to claim 18, wherein the cancer is a blood cancer or solid cancer overexpressing FLT3.

20. The cell therapeutic agent according to claim 18, wherein the hematologic cancer overexpressing FLT3 is acute and chronic leukemia, lymphoma, multiple myelopathy or myelodysplastic syndrome.

21. The cell therapeutic agent according to claim 18, further comprising one or more anticancer agents.

22. The cell therapeutic agent according to claim 21, wherein the anticancer agent is an alkylating agent, antimetabolites, antimicrotubule agents, topoisomerase inhibitors, cytotoxic agents, cell proliferation/migration and survival signaling pathway inhibitors, apoptosis inducers, or histone deacetylase (HDAC) inhibitors.

23. The cell therapeutic agent according to claim 21, wherein the cell proliferation/migration and survival signaling pathway inhibitor is a CXCR4 peptide antagonist.

24. The cell therapeutic agent according to claim 23, wherein CXCR4 peptide antagonist is Plerixafor, BL-8040 or LY2510924.

25. A kit for treating cancer comprising the genetically modified NK cell line of any one claim among claims 1 to 17 and a suicide inducing agent.

26. The kit according to claim 25, wherein the suicide inducing agent is ganciclovir or 6-methoxypurine arabinside when the cell suicide gene is HSV TK or VZV TK, respectively; 5-fluorocytosine (5-FC) when the cell suicide gene is cytosine deaminase (CD) gene, uracil phosphoribosyl transferase (UPRT) gene or a gene encoding a fusion protein of the CD and UPRT (CD::UPRT); irinotecan (CPT-11) when the cell suicide gene is carboxyl esterase; 5-(aziridin-1-yl)-2,4-dinitrobenzamide (CB1954) when the cell suicide gene is nitroreductase; 4-((2-chloroethyl)(2-mesiloxyethyl)amino)benzoyl-L-glutamic acid (CMDA) when the cell suicide gene is carboxypeptidase G2; or an iCas9 dimerizer when cell suicide gene is iCas9.

27. The kit according to claim 26, further comprising one or more anticancer agent.

28. The kit according to claim 27, wherein the anticancer agent is an alkylating agent, antimetabolites, anti-microtubule agents, topoisomerase inhibitors, cytotoxic agents, cell proliferation/migration and survival signaling pathway inhibitors, apoptosis inducers, or histone deacetylase (HDAC) inhibitors.

29. The kit according to claim 28, wherein the cell proliferation/migration and survival signaling pathway inhibitor is a CXCR4 peptide antagonist.

30. The kit according to claim 29, wherein the CXCR4 peptide antagonist is Plerixafor, BL-8040 or LY2510924.

31. A method for treating cancer in an individual in need of cancer treatment, comprising administering a therapeutically effective amount of the genetically modified NK cell line and optionally a suicide inducing agent to the individual

32. The method according to claim 31, comprising additionally performing one or more anticancer therapies selected from stem cell transplantation, radiation therapy, surgical resection, chemotherapy, immunotherapy, targeted therapeutics, or a combination thereof.

33. The method according to claim 31, wherein the suicide inducing agent is ganciclovir or 6-methoxypurine arabinonucleoside when the cell suicide gene is HSV TK or VZV TK, respectively; 5-fluorocytosine (5-FC) when the cell suicide gene is cytosine deaminase (CD) gene, uracil phosphoribosyl transferase (UPRT) gene or a gene encoding a fusion protein of the CD and UPRT (CD::UPRT); irinotecan (CPT-11) when the cell suicide gene is carboxyl esterase; 5-(aziridin-1-yl)-2,4-dinitrobenzamide (CB1954) when the cell suicide gene is nitroreductase; 4-((2-chloroethyl)(2-mesiloxyethyl)amino)benzoyl-L-glutamic acid (CMDA) when the cell suicide gene is carboxypeptidase G2; or an iCas9 dimerizer when cell suicide gene is iCas9.

34. The method according to claim 31, wherein the cancer is a hematologic cancer or solid cancer in which FLT3 is overexpressed.

35. The method according to claim 34, wherein the helamtologic cancer in which FLT3 is overexpressed is acute and chronic leukemia, lymphoma, multiple myelopathy or myelodysplastic syndrome.


<110>    SLBIGEN Inc.

<120>    Genetically engineered NK cell lines transduced with a
         polynucleotide encoding novel chimeric antigen receptor and use
         thereof

<130>    PT20-5207

<150>    KR 10-2019-0115034
<151>    2019-09-18

<160>    26

<170>    KoPatentIn 3.0

<210>    1
<211>    230
<212>    PRT
<213>    Artificial Sequence

<220>
<223>    modified Ig Fc region


<400>    1
Glu Pro Lys Ser Pro Asp Lys Thr His Thr Cys Pro Pro Cys Pro Ser
  1               5                  10                  15

His Thr Gln Pro Leu Gly Val Phe Leu Phe Pro Pro Lys Pro Lys Asp
             20                  25                  30

Gln Leu Met Ile Ser Arg Thr Pro Glu Val Thr Cys Val Val Val Asp
         35                  40                  45

Val Ser Gln Glu Asp Pro Glu Val Gln Phe Asn Trp Tyr Val Asp Gly
     50                  55                  60

Val Glu Val His Asn Ala Lys Thr Lys Pro Arg Glu Glu Gln Phe Asn
 65                  70                  75                  80

Ser Thr Tyr Arg Val Val Ser Val Leu Thr Val Leu His Gln Asp Trp
                 85                  90                  95

Leu Asn Gly Lys Glu Tyr Lys Cys Lys Val Ser Asn Lys Gly Leu Pro
            100                 105                 110

Ser Ser Ile Glu Lys Thr Ile Ser Lys Ala Lys Gly Gln Pro Arg Glu
        115                 120                 125

Pro Gln Val Tyr Thr Leu Pro Pro Ser Gln Glu Glu Met Thr Lys Asn
    130                 135                 140

Gln Val Ser Leu Thr Cys Leu Val Lys Gly Phe Tyr Pro Ser Asp Ile
145                 150                 155                 160

Ala Val Glu Trp Glu Ser Asn Gly Gln Pro Glu Asn Asn Tyr Lys Thr
                165                 170                 175

Thr Pro Pro Val Leu Asp Ser Asp Gly Ser Phe Phe Leu Tyr Ser Arg
            180                 185                 190

Leu Thr Val Asp Lys Ser Arg Trp Gln Glu Gly Asn Val Phe Ser Cys
        195                 200                 205

Ser Val Leu His Glu Ala Leu His Asn His Tyr Thr Gln Lys Ser Leu
    210                 215                 220

Ser Leu Ser Leu Gly Lys
225                 230


<210>    2
<211>    690
<212>    DNA
<213>    Artificial Sequence

<220>
<223>    polynucleotide encoding modified Ig Fc region


<400>    2
gagcccaaat ctcctgacaa aactcacaca tgcccaccgt gcccatccca tacacagcct        60

ctgggagttt ttctttttcc accaaaacca aaggatcagc tgatgatttc ccgcacacct        120

gaagtgacat gcgttgtggt tgatgtttcc caagaagatc ctgaggttca gtttaactgg        180

tacgtggatg gcgtggaggt tcacaatgcc aagaccaagc ctcgcgaaga gcagtttaat        240

agcacctacc gcgttgtgtc cgttcttacc gtgctgcacc aggattggct taatggcaag        300

gagtacaagt gcaaggtgtc aaacaagggc ctgccttcat ccattgagaa gaccatcagc        360

aaggccaagg gacagccaag agaaccacag gtttataccc tgccaccatc ccaagaagag        420

atgacaaaga atcaagttag ccttacctgc cttgtgaagg gcttttaccc tagcgatatt        480

gccgttgagt gggaatccaa tggccagcca gaaaataact acaagaccac acctcctgtt        540

ctggatagcg atggctcctt ctttctgtat tcccgcctta ccgtggataa gagccgctgg        600

caggaaggaa atgttttcag ctgctccgtt ctgcatgagg ctcttcataa ccactacacc        660

cagaagagcc tgtccctcag tcttggcaag                                        690


<210>    3
<211>    27
<212>    PRT
<213>    Artificial Sequence

<220>
<223>    CD28 transmembrane domain


<400>    3
Phe Trp Val Leu Val Val Val Gly Gly Val Leu Ala Cys Tyr Ser Leu
  1               5                  10                  15

Leu Val Thr Val Ala Phe Ile Ile Phe Trp Val
            20                  25


<210>    4
<211>    81
<212>    DNA

<213>    Artificial Sequence

<220>
<223>    polynucleotide encoding CD28 transmembrane domain


<400>    4
ttttgggtgc tggtggtggt tggtggagtc ctggcttgct atagcttgct agtaacagtg        60

gcctttatta ttttctgggt g                                                  81


<210>    5
<211>    22
<212>    PRT
<213>    Artificial Sequence

<220>
<223>    DAP10 intracellular activation domain


<400>    5
Ala Arg Pro Arg Arg Ser Pro Ala Gln Glu Asp Gly Lys Val Tyr Ile
1               5                   10                  15

Asn Met Pro Gly Arg Gly
            20


<210>    6
<211>    66
<212>    DNA
<213>    Artificial Sequence

<220>
<223>    polynucleotide encoding DAP10 intracellular activation domain


<400>    6
gcacgcccac gccgcagccc cgcccaagaa gatggcaaag tctacatcaa catgccaggc        60

aggggc                                                                   66


<210>    7
<211>    49
<212>    PRT
<213>    Artificial Sequence

<220>
<223>    DAP12 intracellular activation domain


<400>    7
Gly Arg Leu Val Pro Arg Gly Arg Gly Ala Ala Glu Ala Ala Thr Arg
1               5                   10                  15

Lys Gln Arg Ile Thr Glu Thr Glu Ser Pro Tyr Gln Glu Leu Gln Gly
            20                  25                  30

Gln Arg Ser Asp Val Tyr Ser Asp Leu Asn Thr Gln Arg Pro Tyr Tyr
        35                  40                  45

Lys

<210> 8
<211> 147
<212> DNA
<213> Artificial Sequence

<220>
<223> polynucleotide encoding DAP12 intracellular activation domain

<400> 8
ggccggctgg tccctcgggg gcgaggggct gcggaggcag cgacccggaa acagcgtatc 60

actgagaccg agtcgcctta tcaggagctc cagggtcaga ggtcggatgt ctacagcgac 120

ctcaacacac agaggccgta ttacaaa 147

<210> 9
<211> 113
<212> PRT
<213> Artificial Sequence

<220>
<223> CD3zeta cytoplasmic domain

<400> 9
Arg Val Lys Phe Ser Arg Ser Ala Asp Ala Pro Ala Tyr Gln Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Gln Arg Arg Lys Asn Pro Gln Glu Gly Leu Tyr Asn Glu Leu Gln
    50                  55                  60

Lys Asp Lys Met Ala Glu Ala Tyr Ser Glu Ile Gly Met Lys Gly Glu
65                  70                  75                  80

Arg Arg Arg Gly Lys Gly His Asp Gly Leu Tyr Gln Gly Leu Ser Thr
                85                  90                  95

Ala Thr Lys Asp Thr Tyr Asp Ala Leu His Met Gln Ala Leu Pro Pro
            100                 105                 110

Arg

<210> 10
<211> 339
<212> DNA
<213> Artificial Sequence

<220>
<223> polynucleotide encoding CD3zeta cytoplasmic domain

<400>    10
agagtgaagt tcagcaggag cgcagacgcc cccgcgtacc agcagggcca gaaccagctc        60

tataacgagc tcaatctagg acgaagagag gagtacgatg ttttggacaa gagacgtggc       120

cgggaccctg agatgggggg aaagccgcag agaaggaaga accctcagga aggcctgtac       180

aatgaactgc agaaagataa gatggcggag gcctacagtg agattgggat gaaaggcgag       240

cgccggaggg gcaaggggca cgatggcctt taccagggcc tcagtacagc caccaaggac       300

acctacgacg cccttcacat gcaggccctg ccccctcgc                              339


<210>    11
<211>    713
<212>    PRT
<213>    Artificial Sequence

<220>
<223>    anti-FLT3 scFv-CAR protein


<400>    11
Met Asp Ala Met Lys Arg Gly Leu Cys Cys Val Leu Leu Leu Cys Gly
1               5                   10                  15

Ala Val Phe Val Ser Pro Ser His Ala Glu Val Gln Leu Val Gln Ser
            20                  25                  30

Gly Ala Glu Val Lys Lys Pro Gly Ser Ser Val Lys Val Ser Cys Lys
        35                  40                  45

Ala Ser Gly Gly Thr Phe Ser Ser Tyr Ala Ile Ser Trp Val Arg Gln
    50                  55                  60

Ala Pro Gly Gln Gly Leu Glu Trp Met Gly Gly Ile Ile Pro Ile Phe
65                  70                  75                  80

Gly Thr Ala Asn Tyr Ala Gln Lys Phe Gln Gly Arg Val Thr Ile Thr
                85                  90                  95

Ala Asp Lys Ser Thr Ser Thr Ala Tyr Met Glu Leu Ser Ser Leu Arg
            100                 105                 110

Ser Glu Asp Thr Ala Val Tyr Tyr Cys Ala Thr Phe Ala Leu Phe Gly
        115                 120                 125

Phe Arg Glu Gln Ala Phe Asp Ile Trp Gly Gln Gly Thr Thr Val Thr
    130                 135                 140

Val Ser Ser Gly Gly Gly Gly Ser Gly Gly Gly Gly Ser Gly Gly Gly
145                 150                 155                 160

Gly Ser Asp Ile Gln Met Thr Gln Ser Pro Ser Ser Leu Ser Ala Ser
                165                 170                 175

Val Gly Asp Arg Val Thr Ile Thr Cys Arg Ala Ser Gln Ser Ile Ser
            180                 185                 190

Ser Tyr Leu Asn Trp Tyr Gln Gln Lys Pro Gly Lys Ala Pro Lys Leu

195                 200                 205

Leu Ile Tyr Ala Ala Ser Ser Leu Gln Ser Gly Val Pro Ser Arg Phe
    210                 215                 220

Ser Gly Ser Gly Ser Gly Thr Asp Phe Thr Leu Thr Ile Ser Ser Leu
225                 230                 235                 240

Gln Pro Glu Asp Leu Ala Thr Tyr Tyr Cys Gln Gln Ser Tyr Ser Thr
                245                 250                 255

Pro Phe Thr Phe Gly Pro Gly Thr Lys Val Asp Ile Lys Ala Ala Ala
            260                 265                 270

Glu Pro Lys Ser Pro Asp Lys Thr His Thr Cys Pro Pro Cys Pro Ser
        275                 280                 285

His Thr Gln Pro Leu Gly Val Phe Leu Phe Pro Pro Lys Pro Lys Asp
    290                 295                 300

Gln Leu Met Ile Ser Arg Thr Pro Glu Val Thr Cys Val Val Val Asp
305                 310                 315                 320

Val Ser Gln Glu Asp Pro Glu Val Gln Phe Asn Trp Tyr Val Asp Gly
                325                 330                 335

Val Glu Val His Asn Ala Lys Thr Lys Pro Arg Glu Glu Gln Phe Asn
            340                 345                 350

Ser Thr Tyr Arg Val Val Ser Val Leu Thr Val Leu His Gln Asp Trp
        355                 360                 365

Leu Asn Gly Lys Glu Tyr Lys Cys Lys Val Ser Asn Lys Gly Leu Pro
    370                 375                 380

Ser Ser Ile Glu Lys Thr Ile Ser Lys Ala Lys Gly Gln Pro Arg Glu
385                 390                 395                 400

Pro Gln Val Tyr Thr Leu Pro Pro Ser Gln Glu Glu Met Thr Lys Asn
                405                 410                 415

Gln Val Ser Leu Thr Cys Leu Val Lys Gly Phe Tyr Pro Ser Asp Ile
            420                 425                 430

Ala Val Glu Trp Glu Ser Asn Gly Gln Pro Glu Asn Asn Tyr Lys Thr
        435                 440                 445

Thr Pro Pro Val Leu Asp Ser Asp Gly Ser Phe Phe Leu Tyr Ser Arg
    450                 455                 460

Leu Thr Val Asp Lys Ser Arg Trp Gln Glu Gly Asn Val Phe Ser Cys
465                 470                 475                 480

Ser Val Leu His Glu Ala Leu His Asn His Tyr Thr Gln Lys Ser Leu
                485                 490                 495

Ser Leu Ser Leu Gly Lys Phe Trp Val Leu Val Val Val Gly Gly Val
            500                 505                 510

Leu Ala Cys Tyr Ser Leu Leu Val Thr Val Ala Phe Ile Ile Phe Trp
        515                 520                 525

Val Ala Arg Pro Arg Arg Ser Pro Ala Gln Glu Asp Gly Lys Val Tyr

530                 535                 540

Ile Asn Met Pro Gly Arg Gly Gly Arg Leu Val Pro Arg Gly Arg Gly
545                 550                 555                 560

Ala Ala Glu Ala Ala Thr Arg Lys Gln Arg Ile Thr Glu Thr Glu Ser
                565                 570                 575

Pro Tyr Gln Glu Leu Gln Gly Gln Arg Ser Asp Val Tyr Ser Asp Leu
            580                 585                 590

Asn Thr Gln Arg Pro Tyr Tyr Lys Arg Val Lys Phe Ser Arg Ser Ala
        595                 600                 605

Asp Ala Pro Ala Tyr Gln Gln Gly Gln Asn Gln Leu Tyr Asn Glu Leu
    610                 615                 620

Asn Leu Gly Arg Arg Glu Glu Tyr Asp Val Leu Asp Lys Arg Arg Gly
625                 630                 635                 640

Arg Asp Pro Glu Met Gly Gly Lys Pro Gln Arg Arg Lys Asn Pro Gln
                645                 650                 655

Glu Gly Leu Tyr Asn Glu Leu Gln Lys Asp Lys Met Ala Glu Ala Tyr
            660                 665                 670

Ser Glu Ile Gly Met Lys Gly Glu Arg Arg Arg Gly Lys Gly His Asp
        675                 680                 685

Gly Leu Tyr Gln Gly Leu Ser Thr Ala Thr Lys Asp Thr Tyr Asp Ala
    690                 695                 700

Leu His Met Gln Ala Leu Pro Pro Arg
705                 710


<210>    12
<211>    2139
<212>    DNA
<213>    Artificial Sequence

<220>
<223>    polynucleotide encoding anti-FLT3 scFv-CAR protein


<400>    12
atggacgcca tgaagagagg cctgtgctgc gtgctgctgc tgtgcggcgc cgtgttcgtg      60

agccccagcc acgccgaggt gcagctggtg cagagcggcg cagaggtgaa gaagccaggc     120

agctccgtga aggtgagctg caaggcctcc ggcggcacct tctctagcta cgccatctcc     180

tgggtgcggc aggcccctgg ccagggcctg gagtggatgg gaggaatcat cccaatcttc     240

ggcacagcca actacgccca gaagtttcag ggcagagtga ccatcacagc cgacaagtcc     300

acctctacag cctatatgga gctgtcctct ctgaggagcg aggataccgc cgtgtactat     360

tgcgccacat tcgccctgtt cggctttcgc gagcaggcct ttgacatctg gggccagggc     420

accacagtga ccgtgagctc cggcggagga ggctccggcg gcggcggctc tggcggcggc     480

ggctccgaca tccagatgac acagagcccc tctagcctga gcgcctccgt gggcgacaga     540

gtgaccatca catgtagagc ctctcagagc atctcctctt acctgaattg gtatcagcag 600

aagcccggca aggcccctaa gctgctgatc tacgcagcca gctccctgca gtctggcgtg 660

cccagccggt tctccggctc tggcagcggc accgacttta ccctgacaat ctctagcctg 720

cagcctgagg atctggccac atactattgt cagcagtcct attctacccc attcacattt 780

ggccccggca ccaaggtgga tatcaaggcg gccgcagagc ccaaatctcc tgacaaaact 840

cacacatgcc caccgtgccc atcccataca cagcctctgg gagtttttct ttttccacca 900

aaaccaaagg atcagctgat gatttcccgc acacctgaag tgacatgcgt tgtggttgat 960

gtttcccaag aagatcctga ggttcagttt aactggtacg tggatggcgt ggaggttcac 1020

aatgccaaga ccaagcctcg cgaagagcag tttaatagca cctaccgcgt tgtgtccgtt 1080

cttaccgtgc tgcaccagga ttggcttaat ggcaaggagt acaagtgcaa ggtgtcaaac 1140

aagggcctgc cttcatccat tgagaagacc atcagcaagg ccaagggaca gccaagagaa 1200

ccacaggttt ataccctgcc accatcccaa gaagagatga caaagaatca agttagcctt 1260

acctgccttg tgaaggcttt ttaccctagc gatattgccg ttgagtggga atccaatggc 1320

cagccagaaa ataactacaa gaccacacct cctgttctgg atagcgatgg ctccttcttt 1380

ctgtattccc gccttaccgt ggataagagc cgctggcagg aaggaaatgt tttcagctgc 1440

tccgttctgc atgaggctct tcataaccac tacacccaga agagcctgtc cctcagtctt 1500

ggcaagtttt gggtgctggt ggtggttggt ggagtcctgg cttgctatag cttgctagta 1560

acagtggcct ttattatttt ctgggtggca cgcccacgcc gcagccccgc ccaagaagat 1620

ggcaaagtct acatcaacat gccaggcagg ggcggccggc tggtccctcg gggcgagggg 1680

gctgcggagg cagcgacccg gaaacagcgt atcactgaga ccgagtcgcc ttatcaggag 1740

ctccagggtc agaggtcgga tgtctacagc gacctcaaca cacagaggcc gtattacaaa 1800

agagtgaagt tcagcaggag cgcagacgcc cccgcgtacc agcagggcca gaaccagctc 1860

tataacgagc tcaatctagg acgaagagag gagtacgatg ttttggacaa gagacgtggc 1920

cgggaccctg agatgggggg aaagccgcag agaaggaaga accctcagga aggcctgtac 1980

aatgaactgc agaaagataa gatggcggag gcctacagtg agattgggat gaaaggcgag 2040

cgccggaggg gcaaggggca cgatggcctt taccagggcc tcagtacagc caccaaggac 2100

acctacgacg cccttcacat gcaggccctg ccccctcgc 2139

<210> 13
<211> 502
<212> PRT
<213> Artificial Sequence

<220>
<223> anti-FLT3 scFv protein

<400>    13
Met Asp Ala Met Lys Arg Gly Leu Cys Cys Val Leu Leu Leu Cys Gly
1               5                   10                  15

Ala Val Phe Val Ser Pro Ser His Ala Glu Val Gln Leu Val Gln Ser
            20                  25                  30

Gly Ala Glu Val Lys Lys Pro Gly Ser Ser Val Lys Val Ser Cys Lys
        35                  40                  45

Ala Ser Gly Gly Thr Phe Ser Ser Tyr Ala Ile Ser Trp Val Arg Gln
    50                  55                  60

Ala Pro Gly Gln Gly Leu Glu Trp Met Gly Gly Ile Ile Pro Ile Phe
65                  70                  75                  80

Gly Thr Ala Asn Tyr Ala Gln Lys Phe Gln Gly Arg Val Thr Ile Thr
                85                  90                  95

Ala Asp Lys Ser Thr Ser Thr Ala Tyr Met Glu Leu Ser Ser Leu Arg
            100                 105                 110

Ser Glu Asp Thr Ala Val Tyr Tyr Cys Ala Thr Phe Ala Leu Phe Gly
        115                 120                 125

Phe Arg Glu Gln Ala Phe Asp Ile Trp Gly Gln Gly Thr Thr Val Thr
    130                 135                 140

Val Ser Ser Gly Gly Gly Gly Ser Gly Gly Gly Gly Ser Gly Gly Gly
145                 150                 155                 160

Gly Ser Asp Ile Gln Met Thr Gln Ser Pro Ser Ser Leu Ser Ala Ser
                165                 170                 175

Val Gly Asp Arg Val Thr Ile Thr Cys Arg Ala Ser Gln Ser Ile Ser
            180                 185                 190

Ser Tyr Leu Asn Trp Tyr Gln Gln Lys Pro Gly Lys Ala Pro Lys Leu
        195                 200                 205

Leu Ile Tyr Ala Ala Ser Ser Leu Gln Ser Gly Val Pro Ser Arg Phe
    210                 215                 220

Ser Gly Ser Gly Ser Gly Thr Asp Phe Thr Leu Thr Ile Ser Ser Leu
225                 230                 235                 240

Gln Pro Glu Asp Leu Ala Thr Tyr Tyr Cys Gln Gln Ser Tyr Ser Thr
                245                 250                 255

Pro Phe Thr Phe Gly Pro Gly Thr Lys Val Asp Ile Lys Ala Ala Ala
            260                 265                 270

Glu Pro Lys Ser Pro Asp Lys Thr His Thr Cys Pro Pro Cys Pro Ser
        275                 280                 285

His Thr Gln Pro Leu Gly Val Phe Leu Phe Pro Pro Lys Pro Lys Asp
    290                 295                 300

Gln Leu Met Ile Ser Arg Thr Pro Glu Val Thr Cys Val Val Val Asp
305                 310                 315                 320

Val Ser Gln Glu Asp Pro Glu Val Gln Phe Asn Trp Tyr Val Asp Gly
                325                 330                 335

Val Glu Val His Asn Ala Lys Thr Lys Pro Arg Glu Glu Gln Phe Asn
            340                 345                 350

Ser Thr Tyr Arg Val Val Ser Val Leu Thr Val Leu His Gln Asp Trp
        355                 360                 365

Leu Asn Gly Lys Glu Tyr Lys Cys Lys Val Ser Asn Lys Gly Leu Pro
    370                 375                 380

Ser Ser Ile Glu Lys Thr Ile Ser Lys Ala Lys Gly Gln Pro Arg Glu
385                 390                 395                 400

Pro Gln Val Tyr Thr Leu Pro Pro Ser Gln Glu Glu Met Thr Lys Asn
                405                 410                 415

Gln Val Ser Leu Thr Cys Leu Val Lys Gly Phe Tyr Pro Ser Asp Ile
            420                 425                 430

Ala Val Glu Trp Glu Ser Asn Gly Gln Pro Glu Asn Asn Tyr Lys Thr
        435                 440                 445

Thr Pro Pro Val Leu Asp Ser Asp Gly Ser Phe Phe Leu Tyr Ser Arg
    450                 455                 460

Leu Thr Val Asp Lys Ser Arg Trp Gln Glu Gly Asn Val Phe Ser Cys
465                 470                 475                 480

Ser Val Leu His Glu Ala Leu His Asn His Tyr Thr Gln Lys Ser Leu
                485                 490                 495

Ser Leu Ser Leu Gly Lys
            500


<210>    14
<211>    264
<212>    PRT
<213>    Artificial Sequence

<220>
<223>    CD7


<400>    14
Met Asp Ala Met Lys Arg Gly Leu Cys Cys Val Leu Leu Leu Cys Gly
1               5                   10                  15

Ala Val Phe Val Ser Pro Ser His Ala Ala Gly Pro Pro Arg Leu Leu
            20                  25                  30

Leu Leu Pro Leu Leu Leu Ala Leu Ala Arg Gly Leu Pro Gly Ala Leu
        35                  40                  45

Ala Ala Gln Glu Val Gln Gln Ser Pro His Cys Thr Thr Val Pro Val
    50                  55                  60

Gly Ala Ser Val Asn Ile Thr Cys Ser Thr Ser Gly Gly Leu Arg Gly
65                  70                  75                  80

Ile Tyr Leu Arg Gln Leu Gly Pro Gln Pro Gln Asp Ile Ile Tyr Tyr

85                  90                  95

Glu Asp Gly Val Val Pro Thr Thr Asp Arg Arg Phe Arg Gly Arg Ile
            100                 105                 110

Asp Phe Ser Gly Ser Gln Asp Asn Leu Thr Ile Thr Met His Arg Leu
        115                 120                 125

Gln Leu Ser Asp Thr Gly Thr Tyr Thr Cys Gln Ala Ile Thr Glu Val
    130                 135                 140

Asn Val Tyr Gly Ser Gly Thr Leu Val Leu Val Thr Glu Glu Gln Ser
145                 150                 155                 160

Gln Gly Trp His Arg Cys Ser Asp Ala Pro Pro Arg Ala Ser Ala Leu
                165                 170                 175

Pro Ala Pro Pro Thr Gly Ser Ala Leu Pro Asp Pro Gln Thr Ala Ser
            180                 185                 190

Ala Leu Pro Asp Pro Pro Ala Ala Ser Ala Leu Pro Ala Ala Leu Ala
        195                 200                 205

Val Ile Ser Phe Leu Leu Gly Leu Gly Leu Gly Val Ala Cys Val Leu
    210                 215                 220

Ala Arg Thr Gln Ile Lys Lys Leu Cys Ser Trp Arg Asp Lys Asn Ser
225                 230                 235                 240

Ala Ala Cys Val Val Tyr Glu Asp Met Ser His Ser Arg Cys Asn Thr
                245                 250                 255

Leu Ser Ser Pro Asn Gln Tyr Gln
            260

<210>    15
<211>    792
<212>    DNA
<213>    Artificial Sequence

<220>
<223>    polynucleotide encoding CD7

<400>    15
atggacgcca tgaagagagg cctgtgctgc gtgctgctgc tgtgcggcgc cgtgttcgtg      60

agccccagcc acgccgccgg ccctcctagg ctgctgctgc tgcctctgct gctggcactg     120

gcccgcgggc tgccaggagc actggccgct caggaggtgc agcagtcccc acactgcacc     180

acagtgcctg tcggagcttc tgtgaacatc acctgttcta caagtggcgg gctgcgagga     240

atctacctgc gacagctggg accccagcca caggacatca tctactatga agatggggtg     300

gtccccacta ccgaccggag attcaggggc cgcatcgact tttcagggag ccaggataac     360

ctgacaatta ctatgcaccg gctgcagctg agtgataccg gcacatacac ttgccaggcc     420

atcaccgaag tgaatgtcta tggcagcggc accctggtgc tggtgacaga ggaacagagc     480

caggggtggc atagatgttc cgacgcacca cccagggctt ctgcactgcc tgctcctcca     540

acaggaagtg cactgcctga cccacagact gcctcagctc tgccagatcc ccctgcagcc 600

agcgccctgc cagctgcact ggctgtgatc agcttcctgc tgggactggg actgggagtg 660

gcatgcgtcc tggcccgcac acagattaag aaactgtgca gttggcgaga caagaacagc 720

gccgcttgcg tggtgtacga agatatgtcc cacagccggt gtaacaccct gagctccccc 780

aatcagtatc ag 792

<210> 16
<211> 244
<212> PRT
<213> Artificial Sequence

<220>
<223> CD28

<400> 16
Met Asp Ala Met Lys Arg Gly Leu Cys Cys Val Leu Leu Leu Cys Gly
1               5                   10                  15

Ala Val Phe Val Ser Pro Ser His Ala Leu Arg Leu Leu Leu Ala Leu
            20                  25                  30

Asn Leu Phe Pro Ser Ile Gln Val Thr Gly Asn Lys Ile Leu Val Lys
        35                  40                  45

Gln Ser Pro Met Leu Val Ala Tyr Asp Asn Ala Val Asn Leu Ser Cys
    50                  55                  60

Lys Tyr Ser Tyr Asn Leu Phe Ser Arg Glu Phe Arg Ala Ser Leu His
65                  70                  75                  80

Lys Gly Leu Asp Ser Ala Val Glu Val Cys Val Val Tyr Gly Asn Tyr
                85                  90                  95

Ser Gln Gln Leu Gln Val Tyr Ser Lys Thr Gly Phe Asn Cys Asp Gly
            100                 105                 110

Lys Leu Gly Asn Glu Ser Val Thr Phe Tyr Leu Gln Asn Leu Tyr Val
        115                 120                 125

Asn Gln Thr Asp Ile Tyr Phe Cys Lys Ile Glu Val Met Tyr Pro Pro
    130                 135                 140

Pro Tyr Leu Asp Asn Glu Lys Ser Asn Gly Thr Ile Ile His Val Lys
145                 150                 155                 160

Gly Lys His Leu Cys Pro Ser Pro Leu Phe Pro Gly Pro Ser Lys Pro
                165                 170                 175

Phe Trp Val Leu Val Val Val Gly Gly Val Leu Ala Cys Tyr Ser Leu
            180                 185                 190

Leu Val Thr Val Ala Phe Ile Ile Phe Trp Val Arg Ser Lys Arg Ser
        195                 200                 205

Arg Leu Leu His Ser Asp Tyr Met Asn Met Thr Pro Arg Arg Pro Gly
    210                 215                 220

Pro Thr Arg Lys His Tyr Gln Pro Tyr Ala Pro Pro Arg Asp Phe Ala
225                 230                 235                 240

Ala Tyr Arg Ser

<210>    17
<211>    732
<212>    DNA
<213>    Artificial Sequence

<220>
<223>    polynucleotide encoding CD28

<400>    17
atggacgcca tgaagagagg cctgtgctgc gtgctgctgc tgtgcggcgc cgtgttcgtg      60

agccccagcc acgccctgag gctgctgctg gccctgaacc tgttccctag catccaggtg     120

acagggaata agattctggt gaaacagtcc ccaatgctgg tcgcatacga caacgccgtg     180

aatctgtctt gcaagtacag ttataacctg ttctcacgag agtttcgggc cagcctgcac     240

aaaggcctgg attctgcagt cgaagtgtgc gtggtctacg ggaattattc ccagcagctg     300

caggtgtact ctaagacagg cttcaactgt gacggaaaac tgggcaatga gagcgtcact     360

ttttacctgc agaacctgta tgtgaatcag accgacatct atttctgtaa gatcgaagtg     420

atgtaccccc ctccatatct ggataacgaa aaatccaatg ggacaatcat tcacgtgaag     480

ggaaaacatc tgtgcccaag tcccctgttc cctggaccat caaagccttt ttgggtcctg     540

gtggtcgtgg gaggggtgct ggcctgttac tctctgctgg tcactgtggc tttcatcatc     600

ttctgggtgc ggagcaagag gtcccgcctg ctgcacagtg actatatgaa catgacccca     660

cggagacccg gccctacacg aaaacattac cagccctatg ccccccctag agatttcgcc     720

gcttacagga gc                                                         732

<210>    18
<211>    22
<212>    PRT
<213>    Artificial Sequence

<220>
<223>    2A self-cleaving peptide

<400>    18
Gly Ser Gly Ala Thr Asn Phe Ser Leu Leu Lys Gln Ala Gly Asp Val
1               5                   10                  15

Glu Glu Asn Pro Gly Pro
            20

<210>    19

<211>    66
<212>    DNA
<213>    Artificial Sequence

<220>
<223>    polynucleotide encoding 2A self-cleaving peptide


<400>    19
ggctccggag ctactaactt cagcctgctg aagcaggctg gagacgtgga ggagaaccct        60

gggccc                                                                    66


<210>    20
<211>    373
<212>    PRT
<213>    Artificial Sequence

<220>
<223>    CD::UPRT protein


<400>    20
Met Val Thr Gly Gly Met Ala Ser Lys Trp Asp Gln Lys Gly Met Asp
1               5                   10                  15

Ile Ala Tyr Glu Glu Ala Ala Leu Gly Tyr Lys Glu Gly Gly Val Pro
            20                  25                  30

Ile Gly Gly Cys Leu Ile Asn Asn Lys Asp Gly Ser Val Leu Gly Arg
        35                  40                  45

Gly His Asn Met Arg Phe Gln Lys Gly Ser Ala Thr Leu His Gly Glu
    50                  55                  60

Ile Ser Thr Leu Glu Asn Cys Gly Arg Leu Glu Gly Lys Val Tyr Lys
65                  70                  75                  80

Asp Thr Thr Leu Tyr Thr Thr Leu Ser Pro Cys Asp Met Cys Thr Gly
                85                  90                  95

Ala Ile Ile Met Tyr Gly Ile Pro Arg Cys Val Val Gly Glu Asn Val
            100                 105                 110

Asn Phe Lys Ser Lys Gly Glu Lys Tyr Leu Gln Thr Arg Gly His Glu
        115                 120                 125

Val Val Val Val Asp Asp Glu Arg Cys Lys Lys Ile Met Lys Gln Phe
    130                 135                 140

Ile Asp Glu Arg Pro Gln Asp Trp Phe Glu Asp Ile Gly Glu Ser Ser
145                 150                 155                 160

Glu Pro Phe Lys Asn Val Tyr Leu Leu Pro Gln Thr Asn Gln Leu Leu
                165                 170                 175

Gly Leu Tyr Thr Ile Ile Arg Asn Lys Asn Thr Thr Arg Pro Asp Phe
            180                 185                 190

Ile Phe Tyr Ser Asp Arg Ile Ile Arg Leu Leu Val Glu Glu Gly Leu
        195                 200                 205

Asn His Leu Pro Val Gln Lys Gln Ile Val Glu Thr Asp Thr Asn Glu
    210                 215                 220

Asn Phe Glu Gly Val Ser Phe Met Gly Lys Ile Cys Gly Val Ser Ile
225                 230                 235                 240

Val Arg Ala Gly Glu Ser Met Glu Gln Gly Leu Arg Asp Cys Cys Arg
                245                 250                 255

Ser Val Arg Ile Gly Lys Ile Leu Ile Gln Arg Asp Glu Glu Thr Ala
            260                 265                 270

Leu Pro Lys Leu Phe Tyr Glu Lys Leu Pro Glu Asp Ile Ser Glu Arg
        275                 280                 285

Tyr Val Phe Leu Leu Asp Pro Met Leu Ala Thr Gly Gly Ser Ala Ile
    290                 295                 300

Met Ala Thr Glu Val Leu Ile Lys Arg Gly Val Lys Pro Glu Arg Ile
305                 310                 315                 320

Tyr Phe Leu Asn Leu Ile Cys Ser Lys Glu Gly Ile Glu Lys Tyr His
                325                 330                 335

Ala Ala Phe Pro Glu Val Arg Ile Val Thr Gly Ala Leu Asp Arg Gly
            340                 345                 350

Leu Asp Glu Asn Lys Tyr Leu Val Pro Gly Leu Gly Asp Phe Gly Asp
        355                 360                 365

Arg Tyr Tyr Cys Val
    370


<210>    21
<211>    1119
<212>    DNA
<213>    Artificial Sequence

<220>
<223>    polynucleotide encoding CD::UPRT protein


<400>    21
atggtgaccg gcggcatggc cagcaagtgg gaccagaagg gcatggacat cgcctacgag      60

gaggccgctc tgggctacaa ggagggcggc gtgcccatcg gcggatgcct gatcaacaac     120

aaggacggca gcgtgctggg cagaggccac aacatgagat tccagaaggg cagcgccacc     180

ctgcacggcg agatcagcac cctggagaac tgcggcagac tggagggcaa ggtgtacaag     240

gacacaaccc tgtacaccac actgagcccc tgcgacatgt gcaccggcgc catcatcatg     300

tacggcatcc ccagatgcgt ggtgggcgag aacgtgaact tcaagagcaa gggcgagaag     360

tacctgcaga ccagaggcca cgaggtggtc gtcgtggacg acgagagatg caagaagatc     420

atgaagcagt tcatcgacga gagaccccag gactggttcg aggacatcgg cgagagcagc     480

gagcccttca agaacgtgta cctgctgccc cagaccaacc agctgctggg cctgtacacc     540

atcatcagaa acaagaacac caccagaccc gacttcatct tctacagcga cagaatcatc     600

agactgctgg tggaggaggg cctgaaccac ctgcccgtgc agaagcagat cgtggagacc 660

gacaccaacg agaacttcga gggcgtgagc ttcatgggca aaatctgcgg cgtgagcatc 720

gtgagagccg gcgagagcat ggagcagggc ctgagagact gctgcagaag cgtgagaatc 780

ggcaagatcc tgatccagag agacgaggag accgccctgc ccaagctgtt ctacgagaag 840

ctgcccgagg acatcagcga gagatacgtg ttcctgctgg accccatgct ggccaccggc 900

ggcagcgcca tcatggccac cgaggtgctg atcaagagag gcgtgaagcc cgagagaatc 960

tacttcctga acctgatctg cagcaaggag ggcatcgaga agtaccacgc tgccttcccc 1020

gaggtgagaa tcgtgaccgg cgccctggac agaggcctgg acgagaacaa gtacctggtg 1080

cccggcctgg gcgacttcgg cgacagatac tactgcgtg 1119

<210> 22
<211> 575
<212> DNA
<213> Artificial Sequence

<220>
<223> internal ribosome entry site (IRES)

<400> 22
cccctctccc ccccctaacg ttactggccg aagccgcttg gaataaggcc ggtgtgcgtt 60

tgtctatatg ttattttcca ccatattgcc gtcttttggc aatgtgaggg cccggaaacc 120

tggccctgtc ttcttgacga gcattcctag gggtctttcc cctctcgcca aaggaatgca 180

aggtctgttg aatgtcgtga aggaagcagt tcctctggaa gcttcttgaa gacaaacaac 240

gtctgtagcg accctttgca ggcagcggaa ccccccacct ggcgacaggt gcctctgcgg 300

ccaaaagcca cgtgtataag atacacctgc aaaggcggca caaccccagt gccacgttgt 360

gagttggata gttgtggaaa gagtcaaatg gctctcctca agcgtattca acaaggggct 420

gaaggatgcc cagaaggtac cccattgtat gggatctgat ctggggcctc ggtgcacatg 480

ctttacatgt gtttagtcga ggttaaaaaa acgtctaggc cccccgaacc acggggacgt 540

ggttttcctt tgaaaaacac gatgataata tggcc 575

<210> 23
<211> 204
<212> PRT
<213> Artificial Sequence

<220>
<223> membrane bound IL-15 (mbIL-15)

<400> 23
Met Ala Leu Pro Val Thr Ala Leu Leu Leu Pro Leu Ala Leu Leu Leu
1               5                   10                  15

His Ala Ala Arg Pro Asn Trp Val Asn Val Ile Ser Asp Leu Lys Lys
            20                  25                  30

Ile Glu Asp Leu Ile Gln Ser Met His Ile Asp Ala Thr Leu Tyr Thr
        35                  40                  45

Glu Ser Asp Val His Pro Ser Cys Lys Val Thr Ala Met Lys Cys Phe
    50                  55                  60

Leu Leu Glu Leu Gln Val Ile Ser Leu Glu Ser Gly Asp Ala Ser Ile
65                  70                  75                  80

His Asp Thr Val Glu Asn Leu Ile Ile Leu Ala Asn Asn Ser Leu Ser
                85                  90                  95

Ser Asn Gly Asn Val Thr Glu Ser Gly Cys Lys Glu Cys Glu Glu Leu
            100                 105                 110

Glu Glu Lys Asn Ile Lys Glu Phe Leu Gln Ser Phe Val His Ile Val
        115                 120                 125

Gln Met Phe Ile Asn Thr Ser Thr Thr Thr Pro Ala Pro Arg Pro Pro
    130                 135                 140

Thr Pro Ala Pro Thr Ile Ala Ser Gln Pro Leu Ser Leu Arg Pro Glu
145                 150                 155                 160

Ala Cys Arg Pro Ala Ala Gly Gly Ala Val His Thr Arg Gly Leu Asp
                165                 170                 175

Phe Ala Cys Asp Ile Tyr Ile Trp Ala Pro Leu Ala Gly Thr Cys Gly
            180                 185                 190

Val Leu Leu Leu Ser Leu Val Ile Thr Leu Tyr Cys
        195                 200


<210>    24
<211>    612
<212>    DNA
<213>    Artificial Sequence

<220>
<223>    polynucleotide encoding membrane bound IL-15 (mbIL-15)


<400>    24
atggccttac cagtgaccgc cttgctcctg ccgctggcct tgctgctcca cgccgccagg      60

ccgaactggg tgaatgtaat aagtgatttg aaaaaaattg aagatcttat tcaatctatg     120

catattgatg ctactttata tacggaaagt gatgttcacc ccagttgcaa agtaacagca     180

atgaagtgct ttctcttgga gttacaagtt atttcacttg agtccggaga tgcaagtatt     240

catgatacag tagaaaatct gatcatccta gcaaacaaca gtttgtcttc taatgggaat     300

gtaacagaat ctggatgcaa agaatgtgag gaactggagg aaaaaaatat taaagaattt     360

ttgcagagtt ttgtacatat tgtccaaatg ttcatcaaca cttctaccac gacgccagcg     420

ccgcgaccac caacaccggc gcccaccatc gcgtcgcagc ccctgtccct gcgcccagag     480

gcgtgccggc cagcggcggg gggcgcagtg cacacgaggg ggctggactt cgcctgtgat     540

atctacatct gggcgccctt ggccgggact tgtggggtcc ttctcctgtc actggttatc     600

accctttact gc                                                         612


<210>  25
<211>  191
<212>  PRT
<213>  Artificial Sequence

<220>
<223>  dominant negative TGFbeta receptor II


<400>  25



<210>  26
<211>  573
<212>  DNA
<213>  Artificial Sequence

<220>
<223>  polynucleotide encoding dominant negative TGFbeta receptor II

<400> 26
atgggtcggg ggctgctcag gggcctgtgg ccgctgcaca tcgtcctgtg gacgcgtatc 60

gccagcacga tcccaccgca cgttcagaag tcggttaata acgacatgat agtcactgac 120

aacaacggtg cagtcaagtt tccacaactg tgtaaatttt gtgatgtgag attttccacc 180

tgtgacaacc agaaatcctg catgagcaac tgcagcatca cctccatctg tgagaagcca 240

caggaagtct gtgtggctgt atggagaaag aatgacgaga acataacact agagacagtt 300

tgccatgacc ccaagctccc ctaccatgac tttattctgg aagatgctgc ttctccaaag 360

tgcattatga aggaaaaaaa aaagcctggt gagactttct tcatgtgttc ctgtagctct 420

gatgagtgca atgacaacat catcttctca gaagaatata acaccagcaa tcctgacttg 480

ttgctagtca tatttcaagt gacaggcatc agcctcctgc caccactggg agttgccata 540

tctgtcatca tcatcttcta ctgctaccgc gtt 573